# EUROPEAN PATENT APPLICATION

(11) **EP 2 172 447 A1**
(43) Date of publication of application: **07.04.2010**
(21) Application number: 08790794.5
(22) Date of filing: 02.07.2008
(51) Int. Cl.: C07C 243/38, A61K 31/195, A61K 31/196, A61K 31/235, A61K 31/245, A61K 31/381, A61K 31/404, A61K 31/416, A61K 31/4192, A61K 31/423, A61K 31/4439, A61K 31/455, A61K 31/4709, A61K 31/538, A61P 3/10, A61P 13/12, A61P 43/00, C07C 233/81, C07C 233/87, C07D 209/08

(54) **AMIDE COMPOUND**

(30) Priority: 03.07.2007 JP 2007174733
(71) Applicant: Astellas Pharma Inc., Tokyo 103-8411 (JP)
(72) Inventor: NOZAWA, Eisuke, Tokyo 103-8411 (JP); IBUKA, Ryotaro, Tokyo 103-8411 (JP); IKEGAI, Kazuhiro, Tokyo 103-8411 (JP); MATSUURA, Keisuke, Tokyo 103-8411 (JP); ZENKOH, Tatsuya, Tokyo 103-8411 (JP); SEO, Ryushi, Tokyo 103-8411 (JP)
(74) Representative: HOFFMANN EITLE
(86) International application number: PCT/JP2008/061943
(87) International publication number: WO 2009/005076

(57) **Abstract**

[Problems] To provide a compound that is useful as an agent for treating chronic renal insufficiency or an agent for treating diabetic nephropathy.

[Means for Solving Problems] The present inventors have conducted extensive studies on a compound having an EP4 receptor antagonistic action, and as a result, they have found that various amide derivatives having a carboxylic group or an equivalent thereof exhibit an excellent EP4 receptor antagonistic action, thereby completing the present invention.

Since the compound of the present invention has an excellent EP4 receptor antagonistic action, it is useful as an agent for preventing and/or treating chronic renal insufficiency or diabetic nephropathy.

## Description

### Technical Field

The present invention relates to a pharmaceutical, in particular, a novel amide compound which is useful as an agent for treating chronic renal insufficiency or diabetic nephropathy.

### Background Art

PGE2 is known as one of the metabolites in an arachidonic acid cascade. The PGE2 exhibits various activities, for example, a pain enhancing action, a pro-inflammatory action, an anti-inflammatory action, an uterine contractile action, a digestive peristalsis promoting action, an awakening action, a gastric acid secretion inhibiting action, a hypotensive action, a platelet aggregation inhibiting action, a bone-resorption promoting action, an angioneogenesis action, and the like.
There are four subtypes, EP1, EP2, EP3 and EP4, for the PGE2 receptors, which have a wide distribution in various tissues. The activation of the EP1 receptor is thought to cause the increase in intracellular Ca²⁺. The EP3 receptor is one of the receptors having different routes for second-messenger systems. The activation of the EP2 and EP4 receptors is thought to cause the activation of an adenylate cyclase, and thus to increase the intracellular cAMP level. In particular, it is thought that the EP4 receptor is associated with the relaxation of smooth muscles, the promotion or inhibition of an inflammatory reaction, the lymphocyte differentiation, the hypertrophy or proliferation of mesangial cells, the secretion of gastrointestinal mucus, and the like.
The inhibitor of a PGE2 receptor, that is, a PGE2 antagonist has a binding activity to the PGE2 receptor. That is, the PGE2 antagonist exhibits a PGE2 antagonistic activity or a PGE2 inhibitory activity. Accordingly, the PGE2 antagonist is expected as a drug for treating the diseases caused by PGE2. Among these, the EP4 receptor antagonist is expected as an agent for treating EP4-related diseases, for example, renal diseases, inflammatory diseases, various pains, and the like, in human and animals. Additionally, the selective antagonist to the EP4 receptor is preferred from the viewpoint that it can avoid the side-effects based on subtypesEP1, EP2, or EP3.

As a compound having an EP4 receptor antagonistic action, for example, the following compound is disclosed in Patent Document 1. 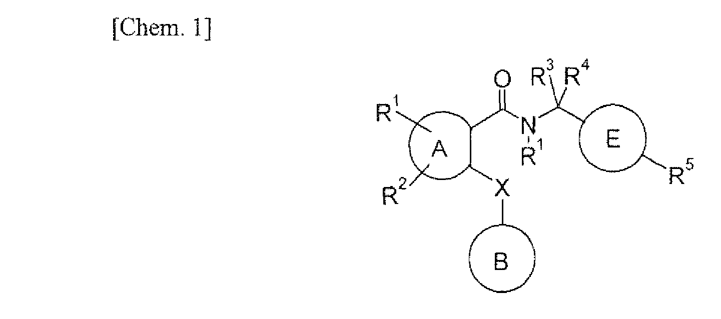 (in the formula, A represents phenyl or pyridyl, E represents 1,4-phenylene, and R⁵ represents -CO₂H, tetrazole, sulfonamide, or carboxylate. For the details, refer to the publication.)
Furthermore, Patent Documents 2 and 3 disclose the compound included in Patent Document 1.

Further, Patent Document 4 discloses the following compound as a compound having an EP4 receptor antagonistic action. 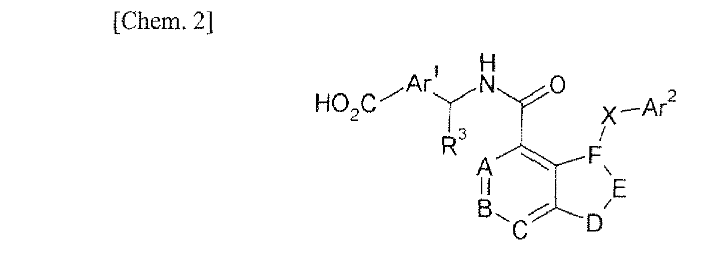 (in the formula, R³ is limited to a group selected from halogen, C₁₋₄alkyl, C₁₋₄fluoroalkyl, C₁₋₄alkoxy, C₁₋₄fluoroalkoxy, and acetyl, and there is no suggestion or disclosure of the compound in which R³ is H. Further, there is no suggestion or disclosure of indole-1,2-diyl or pyrrole-1,2-diyl, and a hydrazine compound. For the details, refer to the publication)

Moreover, Patent Document 6 discloses the following compound as a compound having an EP4 receptor antagonistic action. 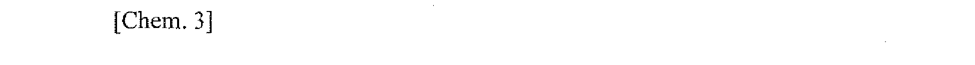 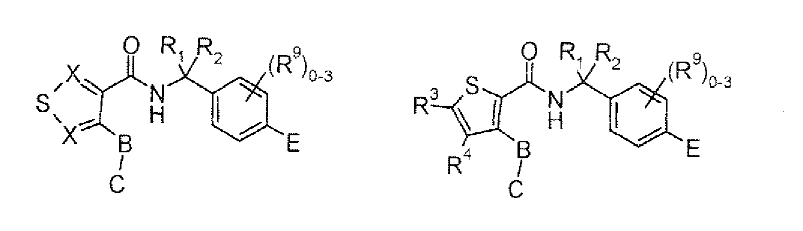 The compound of the Patent Document relates to a thiophene compound, and there is no suggestion or disclosure of an indolyl compound. For the details, refer to the publication.

Patent Document 1: Pamphlet of International Publication No. WO2005/021508
Patent Document 2: Pamphlet of International Publication No. WO2005/1057
Patent Document 3: Pamphlet of International Publication No. WO2005/105733
Patent Document 4: Pamphlet of International Publication No. WO2007/121578
Patent Document 5: Pamphlet of International Publication No. WO2007/143825
Patent Document 6: Pamphlet of International Publication No. WO2008/017164

### Disclosure of the Invention

### Problem that the Invention is to Solve

It is an object of the present invention to provide a novel pharmaceutical having an EP4 receptor antagonistic action, in particular, a novel compound which is useful as an agent for treating chronic renal insufficiency or diabetic nephropathy.

### Means for Solving the Problem

The present inventors have conducted extensive studies on a compound having an EP4 receptor antagonistic action, and as a result, they have found that a compound represented by the following formula (I) exhibits an excellent EP4 receptor antagonistic action, thereby completing the present invention.
Namely, the present invention relates to the compound of the formula (I) or a pharmaceutically acceptable salt thereof: 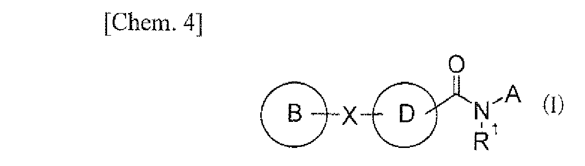 (wherein the symbols in the formula have the following meanings:
Ring B and Ring D: the same as or different from each other, each representing aryl which may be substituted, or a heterocycle which may be substituted,
X: single bond, -O-, -S-, -NH-, -N(R⁰)-, -N(R⁰)-R⁰⁰-, -O-R⁰⁰-, -R⁰⁰-O-, -R⁰⁰-, or -(lower alkenylene)-,
R⁰: lower alkyl,
R⁰⁰: lower alkylene,
R¹: H or R⁰,
A: -Z-R³, or a group represented by any one of the following formulae (II) to (VII): 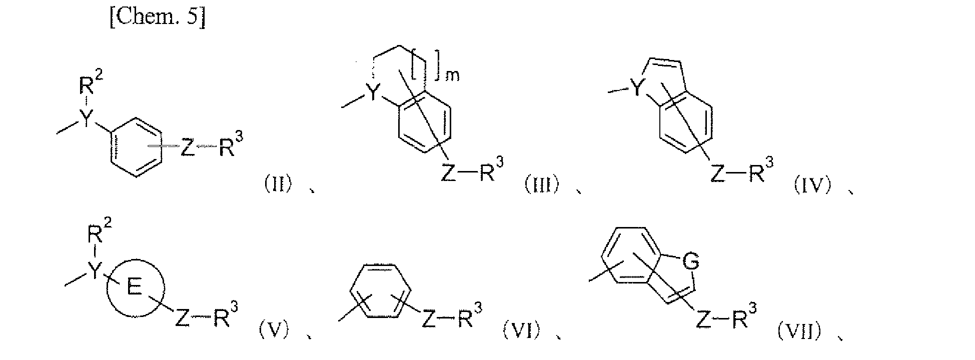
Ring E: a heterocycle or cycloalkanediyl which may be substituted,
Y: CH or N,
R²: H or R⁰,
M: 0 or 1,
Z: single bond or R⁰⁰,
provided that when A is -Z-R³, Z is R⁰⁰,
G: O, S, -N(R²)-, or -(HC=CH)-, and
R³: -CO₂H or a biological equivalent thereof,
provided that the compounds, in which, when D is phenyl or pyridyl which may be substituted, A is a group represented by the above formula (II), Y is CH, and Z is a single bond, R³ is -CO₂H, tetrazole, sulfonamide, or a carboxylate, are excluded).

Additionally, the present application relates to a pharmaceutical composition comprising the compound of the formula (I) or a pharmaceutically acceptable salt thereof as an active ingredient, in particular, relates to an EP4 receptor antagonistic agent or an agent for preventing and/or treating chronic renal insufficiency or diabetic nephropathy.

Further, the present application relates to the use of the compound of the formula (I) or a pharmaceutically acceptable salt thereof, for the manufacture of an agent for treating chronic renal insufficiency or an agent for treating diabetic nephropathy, and to a method for treating chronic renal insufficiency or diabetic nephropathy, which comprises administering to a patient an effective amount of the compound of the formula (I) or a pharmaceutically acceptable salt thereof.

### Effects of Invention

Since the compound of the formula (I) has an EP4 receptor antagonistic action, it is useful as an agent for preventing and/or treating renal diseases, in particular, chronic renal insufficiency, diabetic nephropathy, or the like.

### Best Mode for Carrying out the Invention

Hereinbelow, the present invention will be described in detail.
In the present specification, the "lower alkyl" preferably refers to a linear or branched alkyl having 1 to 6 carbon atoms (which is hereinafter simply referred to as C₁₋₆), and specifically methyl, ethyl, n-propyl, isopropyl, n-butyl, isobutyl, sec-butyl, tert-butyl, n-pentyl, n-hexyl group, or the like. It is more preferably a C₁₋₃ alkyl group, and even more preferably methyl, ethyl, and isopropyl.
The "lower alkylene" means a divalent group (C₁₋₆ alkylene) formed by removing optional one hydrogen atom of the above "lower alkyl", preferably C₁₋₄ alkylene, more preferably C₁₋₃ alkylene, and even more preferably methylene.
The "lower alkenylene" preferably refers to a linear or branched C₂₋₆ alkenylene, and specifically vinylene, ethylidene, propenylene, butenylene, pentenylene, hexenylene, 1,3-butadienylene, 1,3-pentadienylene group, or the like. It is more preferably C₂₋₄ alkenylene, even more preferably ethylidene.
The "halogen" means F, Cl, Br, or I.
The"halogeno-Iower alkyl" refers to C₁₋₆ alkyl substituted with one or more halogen atoms. It is preferably lower alkyl substituted with 1 to 5 halogen atoms, and more preferably trifluoromethyl, 2-fluoroethyl, or 3-fluoropropyl.

The "cycloalkanediyl" means a divalent group (C₃₋₁₀ cycloalkanediyl) formed by removing optional two hydrogen atoms of a C₃₋₁₀ saturated hydrocarbon ring group, and may have a bridge. The bonding position can be any one of 1,1-, 1,2-, 1,3-, 1,4-diyl, or the like, and it is preferably cyclohexanediyl.
The "aryl" refers to a C₆₋₁₄ mono- to tricyclic aromatic hydrocarbon ring group, and includes a partially hydrogenated ring group thereof. Specifically, it is phenyl, naphthyl, 5-tetrahydronaphthyl, 1-indanyl group, or the like. It is preferably phenyl and naphthyl, and more preferably phenyl.

The "heterocyclic group" means a ring group comprising i) a monocyclic 3- to 8- membered, preferably, 5- to 7-membered heterocycle, containing I to 4 hetero atoms selected from oxygen, sulfur, and nitrogen, and ii) a bi- to tricyclic heterocycle containing 1 to 5 hetero atoms selected from oxygen, sulfur, and nitrogen, formed by condensation of the monocyclic heterocycle with one or two rings selected from a group consisting of a monocyclic heterocycle, a benzene ring, C₅₋₈ cycloalkane, and C₅₋₈ cycloalkene. The ring atom, sulfur or nitrogen, may be oxidized to form an oxide or a dioxide.
As the `'heterocyclic group", the following groups may be preferably mentioned:
(1) Monocyclic saturated heterocyclic group
   i) those containing 1 to 4 nitrogen atoms, specifically azepanyl, diazepanyl, aziridinyl, azetidinyl, pyrrolidinyl, imidazolidinyl, piperidinyl, pyrazolidinyl, piperazinyl, and the like;
   ii) those containing 1 to 3 nitrogen atoms and 1 to 2 sulfur atoms and/or I to 2 oxygen atoms, specifically thiomorpholinyl, thiazolidinyl, isothiazolidinyl, oxazolidinyl, morpholinyl group, and the like;
   iii) those containing 1 to 2 sulfur atoms, specifically tetrahydrothienyl group and the like;
   iv) those containing 1 to 2 sulfur atoms and 1 to 2 oxygen atoms, specifically oxathiolane group and the like;
   v) those containing 1 to 2 oxygen atoms, specifically oxiranyl, dioxolanyl, oxolanyl, tetrahydropyranyl, 1,4-dioxanyl group, and the like;
(2) Monocyclic unsaturated heterocyclic group
   i) those containing 1 to 4 nitrogen atoms, specifically pyrrolyl, imidazolyl, pyrazolyl, pyridyl, dihydropyridyl, pyrimidinyl, pyrazinyl, pyridazinyl, triazolyl, tetrazolyl, dihydrotriazinyl, azepinyl group, and the like;
   ii) those containing 1 to 3 nitrogen atoms and 1 to 2 sulfur atoms and/or 1 to 2 oxygen atoms, specifically thiazolyl, isothiazolyl, thiadiazolyl, dihydrothiazinyl, oxazolyl, isooxazolyl, oxadiazolyl, oxadinyl group, and the like;
   iii) those containing 1 to 2 sulfur atoms, specifically thienyl, thiepinyl, dihydrodithienyl, dihydrodithionyl group, and the like;
   iv) those containing 1 to 2 sulfur atoms and 1 to 2 oxygen atoms, specifically dihydrooxathiinyl group, and the like;
   v) those containing 1 to 2 oxygen atoms, specifically furyl, pyranyl, oxepinyl, dioxolyl group, and the like;
(3) Condensed polycyclic saturated heterocyclic group
   i) those containing 1 to 5 nitrogen atoms, specifically quinuclidine, 7-azabicyclo[2.2.1]heptyl, 3-azabicyclo[3.2.2]nonanyl group, and the like;
   ii) those containing 1 to 4 nitrogen atoms, and 1 to 3 sulfur atoms and/or 1 to 3 oxygen atoms, specifically trithiadiazaindenyldioxoloimidazolidinyl group, and the like;
   iii) those containing 1 to 3 sulfur atoms and/or 1 to 3 oxygen atoms, specifically 2,6-dioxabicyclo[3.2.2]oct-7-yl group, and the like;
(4) Condensed polycyclic unsaturated heterocyclic group
   i) those containing 1 to 5 nitrogen atoms, specifically indolyl, isoindolyl, indolinyl, indolidinyl, benzoimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, quinoxalinyl, dihydroindazolyl, benzopyrimidinyl, naphthyridinyl, quinazolinyl, cinnolinyl group, and the like;
   ii) those containing 1 to 4 nitrogen atoms, and 1 to 3 sulfur atoms and/or 1 to 3 oxygen atoms, specifically benzothiazolyl, dihydrobenzothiazolyl, benzothiadiazolyl, imidazothiazolyl, imidazothiadiazolyl, benzooxazolyl, benzooxadiazolyl group, and the like;
   iii) those containing 1 to 3 sulfur atoms, specifically benzothienyl, benzodithiinyl group, and the like;
   iv) those containing 1 to 3 sulfur atoms and 1 to 3 oxygen atoms, specifically benzooxathiinyl, phenoxadinyl group, and the like;
   v) those containing 1 to 3 oxygen atoms, specifically benzodioxolyl, benzofuranyl, isobenzofuranyl, chromenyl, benzodihydrofuranyl group, and the like.

The above "aryl" and "heterocycle" are described as monovalent groups, but in the case where such a group is described as a bi- or higher-valent group, for example, as in the case of Ring D, they each represent a group formed by removing hydrogen atoms at optional positions.

The "-CO₂H or a biological equivalent thereof" means carboxylic acid, or another atom or atom group, which has an electronic configuration or stereochemistry equivalent to carboxylic acid and has common biological properties. These are so-called carboxylic acid bioisostere, a protected carboxylic group, a prodrug of a carboxylic acid, and the like, which are usually used by a skilled person in the art, for example, carboxylic acid, carboxylate, hydroxamic acid (R-CO-NH-OH), sulfonamide (R-NH-SO₂-R'), acylcyanamide (R-CO-NH-CN), acylsulfonamide (R-CO-NH-SO₂-R'), or tetrazole, oxadiazolone, oxadiazolthione, oxathiadiazole, thiadiazolone, triazolthione, hydroxyisoxazole group, and the like, and preferably carboxylic acid and oxadiazolone.
The "protected carboxylic" group can include the following groups:
(1) Esterified carboxylic group: specifically, -CO-O-R⁰, -CO-O-(lower alkenyl), -CO-O-(lower alkynyl), -CO-OR⁰⁰-O-R⁰, -CO-O-R⁰⁰-(aryl), -CO-O-R⁰⁰-O-(aryl) group, or the like; and
(2) Amidated carboxylic group: specifically, -CO-NH₂, -CO-NH-R⁰, -CO-N(R⁰)₂, -CO-N(R⁰)-(aryl), -CO-N(R⁰)-R⁰⁰-(aryl), -CO-NH-R⁰⁰-OH, -CO-NH-R⁰⁰-CO₂H group, or the like.

The term "which may be substituted" represents "which is not substituted" or "which is substituted with 1 to 5 substituents which are the same as or different from each other". Further, if it has a plurality of substituents, the substituents may be the same as or different from each other.
The substituent for the "aryl which may be substituted" and the "heterocycle which may be substituted" is preferably a group selected from -R⁰, halogeno-lower alkyl, -OH, -OR⁰, halogen, oxo, -NO₂,-CN, and -S(O)₂-R⁰, and more preferably a group selected from -R⁰, halogen, and oxo.
The "selective" in the "the selective antagonistic action to the EP4 receptor" means that the affinity to the EP4 is higher than the other three subtypes among the four subtypes EP1, EP2, EP3, and EP4 of the PGE2 receptor. The difference in the affinities is preferably 5-fold, more preferably 10-fold, and even more preferably 100-fold or more.

Preferred embodiments of the substituent of the compound of the formula (I) will be described below.
(1) Ring B is preferably phenyl, thienyl, pyridyl or quinolyl, which may be each substituted with a group selected from the group consisting of R⁰, halogen, halogeno-lower alkyl, -C)R⁰, and -S(O)₂R⁰, more preferably phenyl which may be each substituted with halogen or -OR⁰, even more preferably chlorophenyl, and still more preferably 4-chlorophenyl.
(2) Ring D is preferably a monocyclic or bicyclic heterocycle, which may be each substituted with halogen or R⁰, more preferably a bicyclicheterocycle, which may be each substituted with halogen or R⁰, even more preferably indolyl, indolinyl, indazolyl, benzooxadinyl, benzooxazolyl, or benzotriazolyl, which may be each substituted with halogen, and still more preferably indolyl, which may be substituted with C1.
   In another preferred embodiment, Ring D is phenyl, pyridyl, pyrrolyl, or thienyl, which may be each substituted with halogen or R⁰. Further, in a still further preferred embodiment, Ring D is indolyl, which may be substituted with halogen or R⁰, and in a still further preferred embodiment, it is indole-1,2-diyl or pyrrole-1,2-diyl, which may be each substituted with halogen or R⁰.
(3) X is preferably -O-, methylene, or -R⁰⁰-O-, and more preferably methylene.
(4) A is preferably a group represented by the following formula (II), (III), or (V), 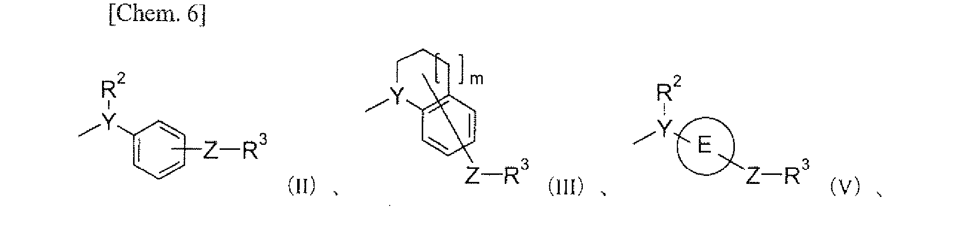 more preferably a group represented by the formula (II). Further, in a above preferred embodiment, A is a group represented by any one of the formula (II) and the formula (V).
(5) R² is preferably H or C₁₋₃ alkyl, and even more preferably methyl.
(6) Z is preferably a single bond.
(7) R³ is preferably -CO₂H.
(8) Y is preferably CH, and in another preferred embodiment, N.
(9) R² is preferably H, and in another preferred embodiment, R⁰.

The embodiments of the compound of the formula (I) are the compound formed from the combinations of the preferable groups as described in (I) to (9) above, and include the following compounds or pharmaceutically acceptable salts thereof.
(10) The compound, wherein Ring B is phenyl, thienyl, pyridyl, or quinolyl, which may be each substituted with a group selected from the group consisting of R⁰, halogen, halogeno-lower alkyl, -OR⁰, and -S(O)₂R⁰, Ring D is a bicyclicheterocycle, which may be each substituted with halogen or R⁰, X is -O-, methylene, or -R⁰⁰-O-, and A is a group represented by the formula (II), (III) or (V).
(11) The compound, wherein Ring B is phenyl, which may be each substituted with halogen or -OR⁰, Ring D is phenyl, pyridyl, pyrrolyl, thienyl, indolyl, or indolinyl, which may be each substituted with halogen, X is methylene, A is a group represented by the formula (II), R² is H or C₁₋₃ alkyl, Z is a single bond, and R³ is a carboxylic acid.
(12) The compound as described in (11) above, wherein Ring B is 4-chlorophenyl, Ring D is indolyl which may be substituted with halogen, and R² is methyl.
(13) The compound, wherein Ring D is a bicyclicheterocycle, which may be substituted with halogen or R⁰, A is a group represented by the formula (II) or the formula (V), Y is CH, and R² is H.
(14) The compound, wherein Ring D is indolyl, which may be substituted with halogen or R⁰, A is a group represented by the formula (II) or the formula (V), Y is CH, and R² is H.
(15) The compound, wherein Ring D is indolyl, which may be substituted with halogen or R⁰, and Ring E is cyclohexanediyl.
(16) The compound, wherein Ring D is indole-1,2-diyl or pyrrole-1,2-diyl, which may be substituted with halogen or R⁰.
(17) The compound, wherein A is a group represented by any one of the formulae (II) to (V), and Y is N.

The compound of the formula (I) may exist in the form of other tautomers or geometrical isomers in some cases, depending on the kinds of the substituents. In the present specification, the compound may be described in only one form of such isomers, but the present invention includes such isomers, isolated forms of the isomers, or a mixture thereof.
Furthermore, the compound of the formula (I) may have asymmetric carbon atoms or axial asymmetries in some cases, and correspondingly, it may exist in the form of optical isomers such as an (R)-form, an (S)-form, and the like. The present invention includes both a mixture and an isolated form of these optical isomers.
Additionally, the pharmaceutically acceptable prodrugs of the compound of the formula (I) are also included in the present invention. The pharmaceutically acceptable prodrug refers to a compound having a group which can be converted into an amino group, OH, CO₂H, or the like, by solvolysis or under a physiological condition. Examples of the groups for forming a prodrug include those as described in Prog. Med., 5, 2157-2161 (1985) or "Pharmaceutical Research and Development" (Hirokawa Publishing Company, 1990), vol. 7, Drug Design, 163-198.

Furthermore, the compound of the formula (I) may form an acid addition salt or a salt with a base, depending on the kind of the substituents, and these salts are included in the present invention, as long as they are pharmaceutically acceptable salts. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid, and the like, and salts with inorganic bases such as sodium, potassium, magnesium, calcium, aluminum, and the like, and organic bases such as methylamine, ethylamine, ethanolamine, lysine, ornithine, and the like, ammonium salts, and others.
Additionally, the present invention also includes various hydrates or solvates, and polymorphism of the compound of the formula (I) and a pharmaceutically acceptable salt thereof. Furthermore, the present invention also includes the compounds labeled with various radioactive isotopes or non-radioactive isotopes.

### (Production Processes)

The compound of the formula (I) and a pharmaceutically acceptable salt thereof can be prepared by applying various known synthetic methods, using the characteristics based on their basic skeletons or the kinds of the substituents. At this time, depending on the types of the functional groups, it is in some cases effective from the viewpoint of the preparation techniques to substitute the functional group with an appropriate protecting group (a group which is capable of being easily converted into the functional group), during the steps from starting materials to intermediates. Examples of such functional groups include an amino group, a hydroxyl group, a carboxyl group, and the like, and examples of the protecting group thereof include those as described in "Protective Groups in Organic Synthesis (3rd edition, 1999)", edited by Greene and Wuts, and the like, which may be appropriately selected and used depending on the reaction conditions. In these methods, a desired compound can be obtained by introducing the protecting group to carry out the reaction, and then, if desired, removing the protecting group.
Additionally, the prodrug of the compound of the formula (I) can be prepared by introducing a specific group during the steps from starting materials to intermediates, in the same manner as for the above protecting groups, or by carrying out the reaction using the obtained compound (I). The reaction can be carried out by applying a method known by a person skilled in the art, such as general esterification, amidation, dehydration, and the like.
Hereinbelow, the representative production processes for the compound of the formula (I) will be described. Each production process may also be carried out with reference to the References appended in the present description. Further, the production processes of the present invention are not limited to the examples as shown below.

### (Production Process 1)

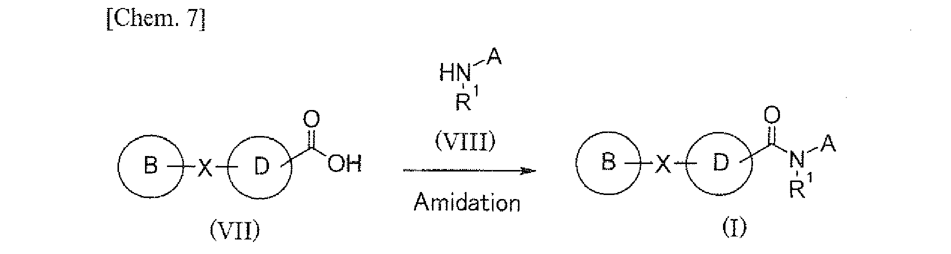

The present production process is a method for preparing the compound of the formula (I) by reacting a compound (VII) with a compound (VIII).
The reaction is carried out using the compound (VII) and the compound (VIII) in equivalent amounts or either thereof in an excessive amount from under cooling to under heating, preferably at -20°C to 60°C, usually by stirring for 0.1 hour to 5 days in a solvent which is inert to the reaction, in the presence of a condensing agent. Here, the solvent is not particularly limited, but, aromatic hydrocarbons such as benzene, toluene, xylene, and the like, halogenated hydrocarbons such as dichloromethane (DCM), 1,2-dichloroethane (DCE), chloroform, and the like, ethers such as diethyl ether, tetrahydrofuran (THF), dioxane, dimethoxyethane (DME), and the like, N,N-dimethylformamide (DMF), dimethylsulfoxide (DMSO), ethyl acetate, acetonitrile, or water, or a mixture thereof. As the condensing agent, 1-[bis(dimethylamino)methylene]-1H-1,2,3-triazolo[4,5-b]pyridin-1-ium-3-oxide hexafluorophosphate (HATU), 1-(3-dimethylaminopropyl)-3-ethylcarbodiimide (WSC), dicyclohexylcarbodiimide (DCC), 1,1'-carbonyldiimidazole (CDI), diphenylphosphoryl azide, phosphorous oxychloride, a condensing agent-carrying polystyrene resin, for example, a PS-carbodiimide (Argonaut Technologies, Inc., USA), or the like may be preferably used in some cases, but are not limited thereto. It may be preferable in some cases for the reaction to use an additive (for example, 1-hydroxybenzotriazole (HOBt) and the like), and it may be advantageous in some cases for the smooth progress of the reaction to carry out the reaction, for example, in the presence of an organic base such as triethylamine, N,N-diisopropylethylamine (DIPEA), N-methylmorpholine, and the like, or an inorganic base such as potassium carbonate, sodium carbonate, potassium hydroxide, and the like. Also, it is preferable to use an isocyanate-carrying polystyrene resin, for example, PS-Isocyanate (Argonaut Technologies, Inc., USA) and the like, in order to remove an excessive amine after completion of the reaction. Additionally, it may be advantageous in some cases to use a quaternary ammonium salt-carrying polystyrene resin, for example, MP-Carbonate (Argonaut Technologies, Inc., USA) and the like, in order to remove excessive carboxylic acid and the afore-mentioned additives, and the like after completion of the reaction.
Furthermore, a method in which the compound (VII) is derived into a reactive derivative thereof, and reacted with the compound (VIII) can also be used. Here, examples of the reactive derivative of the compound (VII) include acid halides obtained by the reaction of a halogenating agent such as phosphorous oxychloride, thionyl chloride, and the like, mixed acid anhydrides such as isobutyl chloroformate, and the like, active esters obtained by condensation with HOBt, and the like, and others. The reaction of the reactive derivative and the compound (VIII) can be carried out, from under cooling to under heating, preferably at -20°C to 60°C, in a solvent which is inert to the reaction, such as halogenated hydrocarbons, aromatic hydrocarbons, ethers, and the like.

### (Production Process 2)

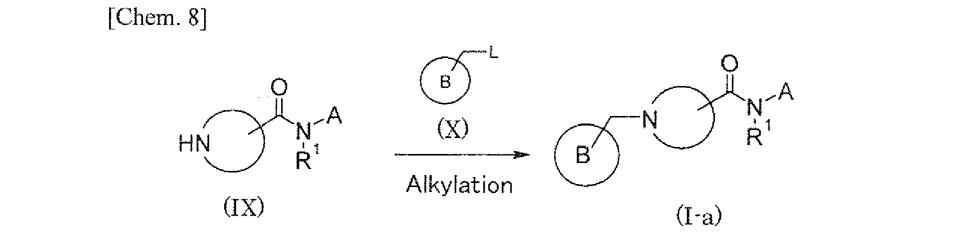

(in the formula, L represents a leaving group. The same shall apply hereinafter.)
The present production process is a method for obtaining the compound (I-a) of the present invention by reacting a compound (IX) and a compound (X). Here, examples of the leaving group include halogen, methanesulfonyloxy, p-toluenesulfonyloxy group, and the like.
The reaction is carried out using the compound (IX) and the compound (X) in equivalent amounts or either thereof in an excessive amount from under cooling to under heating under reflux, preferably at 0°C to 80°C, usually by stirring for 0.1 hour to 5 days in a solvent which is inert to the reaction or without a solvent. Here, the solvent is not particularly limited, but examples thereof include aromatic hydrocarbons, ethers, halogenated hydrocarbons, DMF, DMSO, ethyl acetate, acetonitrile, or a mixture thereof.
It may be advantageous in some cases for the smooth progress of the reaction to carry out the reaction in the presence of organic bases such as triethylamine, DIPEA, N-methylmorpholine, and the like, or inorganic bases such as potassium carbonate, sodium carbonate, potassium hydroxide, and the like.
Furthermore, by subjecting the compound of the formula (I) in which R³ is a carboxylate to hydrolysis under alkali or deprotection of the carboxylic acid, the compound of the formula (I) in which R³ is a carboxylic acid can be obtained.

### (Starting Material Synthesis)

### Starting Material Production Process 1

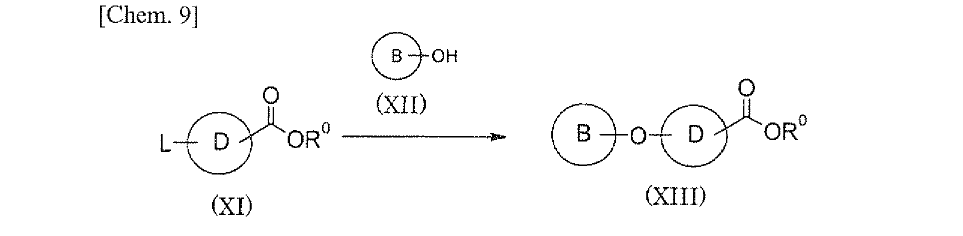

The compound (XIII) can be prepared by reacting a compound (XI) and a compound (XII). The reaction is carried out from under cooling to under heating under reflux, preferably at 0°C to 80°C, usually by stirring for 0.1 hour to 5 days, in a solvent which is inert to the reaction, in the presence of a base. Here, the solvent is not particularly limited, but examples thereof include aromatic hydrocarbons, ethers, halogenated hydrocarbons, DMF, DMSO, ethyl acetate, acetonitrile, or a mixture thereof. Examples of the base include organic bases such as triethylamine, DIPEA, 1,8-diazabicyclo[5.4.0]-7-undecene, n-butyl lithium, and the like, and inorganic bases such as sodium carbonate, potassium carbonate, sodium hydride, potassium tert-butoxide, and the like. Also, it may be preferable in some cases that the present reaction is carried out in the presence of a phase transfer catalyst such as tetra-n-butylammonium chloride and the like, a copper-copper iodide catalyst, and others.
By hydrolyzing the resulting compound (XIII), a corresponding carboxylic acid can be prepared.

### Starting Material Production Process 2

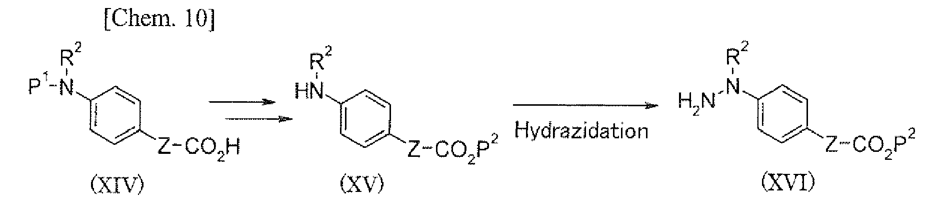

(P¹ and P² each represent an amino group and the protecting group of a carboxylic acid. P¹ is preferably a t-butoxycarbonyl group, and P² is preferably C₁₋₆ alkyl or allyl. The same shall apply hereinafter.)
The compound (XVI) can be prepared by the hydrazidation of the compound (XV) obtained by protecting the carboxylic acid of the compound (XIV), and then deprotecting the amino group.

The compound of the formula (I) is isolated and purified as a free compound, pharmaceutically acceptable salts thereof, hydrates, solvates, or polymorphism thereof.
The pharmaceutically acceptable salt of the compound of the formula (I) can also be prepared in accordance with a conventional method for a salt formation reaction.
Isolation and purification are carried out by employing general chemical operations such as extraction, fractional crystallization, various types of fractional chromatography, and the like.
Various isomers can be separated by selecting an appropriate starting compound or by making use of the difference in the physicochemical properties between isomers.
For example, the optical isomer can be lead into a stereochemically pure isomer by means of general optical resolution methods (for example, fractional crystallization for inducing diastereomer salts with optically active bases or acids, chromatography using a chiral column and the like, and others). Additionally, the isomers can also be prepared from an appropriate optically active starting compound.

The pharmacological activity of the compound of the formula (I) was confirmed by the following test.
Test Example 1: Evaluation test on rat EP4 receptor affinity
Cell culture and transfection
Using a 10 cm collagen-coated dish (Asahi Glass), HEK293 cells were cultured in a D-MEM culture medium, the culture medium was removed at a confluence (90 to 100% density state) and washed with a phosphate-buffered saline (PBS), and then the cells were detached with N,N,N,N'-tetrakis(carboxymethyl)ethylenediamine (EDTA). The number of the cells were counted and seeded on a 15 cm collagen-coated dish to a confluence of 70%. The next day, to an Opti-MEM culture medium at 1.2 mL/dish was added Lipofectamine 2000 (Invitrogen) at 60 µL/dish, followed by being left to stand at room temperature for 5 minutes. A plasmid in which a rat EP4 (sequence number 1) had been inserted into a TA cloning site of pcDNA3.1-V5-His-topo was added thereto at 15 µg/dish. After leaving it to stand at room temperature for 30 minutes, the resultant was added to the dish and cultured for 20 to 24 hours. The cell culture was carried out in a CO₂ incubator (37°C, 5% CO₂).
Preparation of membrane fraction
The culture medium was removed by suction, 10 mL of cooled PBS was added thereto per 15 cm dish, and the cells were scraped using a cell scraper (Sumitomo Bakelite). After washing with cooled PBS (1,200 rpm, 4°C, 5 min), and then suspended in 6 mL of cooled 20 mM Tris-HCl (pH 7.4; Nakalai Tesque Inc., including 5 mM EDTA (Nakalai Tesque Inc.) per dish and homogenized using a Polytron, the homogenate was centrifuged (26,000 rpm, 20 min, 4°C). The obtained precipitate was resuspended in cooled 20 mM Tris-HCl and homogenized again using a Polytron, and the homogenate was centrifuged (26,000 rpm, 20 min, 4°C). The obtained precipitate was resuspended in 50 mM HEPES (pH 7.5; Dojindo Laboratories) to 1 mL per dish, homogenized using a Polytron, and freeze-stored at -80°C as a membrane fraction. At this time, a part thereof was used for the measurement of the protein concentration. Measurement of the protein concentration was carried out using a Bio-Rad Protein assay kit (Bio-Rad Laboratories) in accordance with the appended standard Protocol in duplicate.
Binding Assay
[³H] PGE2 50 µL (final concentration 0.3 nM; Perkin Elmer), 100 µL(20 µg/well) of a membrane fraction prepared from the rat EP4 expression cell and 50 µL of a test compound were mixed in a 96-well microplate (Sumitomo Bakelite), incubated at room temperature for I hour, filtered by suction on a UniFilter-96 GF/B (Perkin Elmer) using a FilterMate Harvester (Perkin Elmer), and then washed three times with 300 µL/well of a cooled assay buffer. Dilution of [³H]PGE2 and the membrane fraction was carried out using an assay buffer (50 mM HEPES, 10 mM MgCl₂), and dilution of the test compound and the unlabeled PGE2 was carried out using DMSO and the assay buffer. Further, in the case a human serum albumin (HSA) is added, dilution was carried out using an assay buffer containing 4% HSA (final concentration 1%; Sigma). The UniFilter-96 GF/B was treated by preliminarily washing twice with 200 µL/well of a cooled assay buffer. The UniFilter-96 GF/B after filtration was dried in a dryer overnight, 50 µL/well of MicroScint20 (Perkin Elmer) was added thereto, and then the radioactivity was measured using a TopCount (Perkin Elmer). For measurement of the non-specific binding, an unlabeled PGE2 (final concentration 1 µM; Cayman) was added. All of the measurements were carried out in duplicate, and the specific binding amount was determined by subtracting the non-specific binding amount from the total binding amount.

According to Test method as above, the rat EP4 receptor affinity (Ki) of the compound of the formula (I) was measured. The Ki values of the representative Example compounds of the present invention are shown in Table 1 below. Ex represents Example Compound number.

**[Table 1]**

| Ex | Ki \|nM\| |
|---|---|
| 4 | 7.5 |
| 7 | 16 |
| 8 | 0.8 |
| 15 | 3.5 |
| 21 | 0.51 |
| 22 | 0.79 |
| 31 | 0.83 |
| 37 | 9.9 |
| 39 | 37 |
| 46 | 11 |
| 47 | 5.5 |
| 50 | 15 |
| 57 | 1.6 |
| 61 | 12 |
| 110 | 12 |
| 111 | 2.6 |

Furthermore, as a result of evaluating the compound of Example 51 of Patent Document 3 according to the test method as above, it was found that the rat EP4 receptor affinity (Ki) of the compound was 48 nM.

Test Example 2: Test to investigate the effect on urine albumin in Streptozotocin (STZ)-induced diabetic rats.
Eight-week old male Wistar (Crj) rats were divided into groups with unbiased urinary albumin excretion (UAE), and STZ (50 mg/kg) was intravenously administered thereto. From the next day of administration of STZ, the drug was continuously orally administered, and urine was periodically collected in a metabolism cage for 24 hours to measure the UAE.

As a result of each of the above tests, it was confirmed that the compound of the present invention is useful as a pharmaceutical, in particular, an agent for preventing and/or treating various diseases involving EP4 as below. It can be used, for example, as an agent for treating renal diseases (for example, acute nephritis, recurrent hematuria, persistent hematuria, chronic nephritis, rapidly progressive nephritis, acute renal insufficiency, chronic renal insufficiency, diabetic nephropathy, Bartter's syndrome, and the like), inflammatory skin diseases (for example, sunburn, bums, eczema, dermatitis, and the like), ischemic heart diseases caused by arteriosclerosis (especially, myocardial infarction, angina, and the like), cerebrovascular disorders caused by arteriosclerosis (stroke, strokes including stroke and lacunar infarction, cerebral thrombosis, cerebral hemorrhage, subarachnoid hemorrhage, cerebral infarction, and the like), peptic ulcer (gastric ulcer, duodenal ulcer, and the like), malignant tumor and metastasis thereof (colon cancer, breast cancer, and the like), and the like, or the analogous diseases in humans and animals, in particular, renal diseases such as chronic renal insufficiency, diabetic nephropathy, and the like.
The compound of the formula (I) or a salt thereof is useful as a pharmaceutical preparation having a diuretic effect. Those having a diuretic effect are useful as a pharmaceutical for treating or preventing various types of edema (for example, cardiac edema, cerebral edema, and the like), hypertension such as malignant hypertension, and the like, a premenstrual syndrome, a poor urine disease caused by urinary calculi, an acute or chronic disease, hyperphosphatemia, and the like.

A preparation containing one or two or more kinds of the compound of the formula (I) or a salt thereof as an active ingredient can be prepared in accordance with a generally used method, using a pharmaceutical carrier, excipient, or the like, that is usually used in the art.
The administration can be carried out in any form of oral administration via tablets, pills, capsules, granules, powders, liquid preparations, or the like; or parenteral administration via injections such as intraarticular, intravenous, or intramuscular injections, suppositories, eye drops, eye ointments, percutaneous liquid preparations, ointments, percutaneous patches, transmucosal liquid preparations, transmucosal patches, inhalations, and the like.

As the solid composition for oral administration according to the present invention, tablets, powders, granules, or the like are used. In such a solid composition, one or two or more kinds of active ingredients are mixed with at least one inert excipient such as lactose, mannitol, glucose, hydroxypropylcellulose, microcrystalline cellulose, starch, polyvinyl pyrrolidone, and/or magnesium aluminometasilicate. According to a conventional method, the composition may contain inert additives such as a lubricant such as magnesium stearate, a disintegrator such as carboxymethylstarch sodium, a stabilizing agent, and a solubilizing agent. As occasion demands, the tablets or the pills may be coated with a sugar coating, or a film of a gastric or enteric material.
The liquid composition for oral administration includes pharmaceutically acceptable emulsions, solutions, suspensions, syrups, elixirs, or the like, and contains a generally used inert diluent such as purified water or ethanol. In addition to the inert diluent, this liquid composition may contain an auxiliary agent such as a solubilizing agent, a moistening agent, and a suspending agent, a sweetener, a flavor, an aroma, and an antiseptic.
The injections for parenteral administration include sterile aqueous or non-aqueous liquid preparations, suspensions and emulsions. The aqueous solvent includes, for example, distilled water for injection and physiological saline. Examples of the non-aqueous solvent include propylene glycol, polyethylene glycol, plant oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent. These are sterilized, for example, by filtration through a bacteria retaining filter, blending of a bactericide, or irradiation. Additionally, these can also be used by preparing a sterile solid composition, and dissolving or suspending it in sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.
As the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, and the like, or other forms.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. Additionally, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compound of the formula (I) can be used in combination with various agents for treating or preventing the diseases for which the compound of the present invention is considered to be effective. The combined preparation may be administered simultaneously, or separately and continuously or at a desired time interval. The preparations to be co-administered may be a blend, or may be prepared individually.

### Examples

Hereinbelow, the production processes of the compound of the formula (I) are described in more detail with reference to Examples. The compounds of the formula (I) are not limited to the compounds as described in Examples below. Additionally, the production processes of the starting compounds are shown in the Production Examples.

Production Example 1 To a mixture of 2,5-dichloronicotinie acid (0.99 g) and DMF (15 mL) were added 3,4-difluorophenol (0.80 g), potassium carbonate (1.6 g), copper (0.072 g), and copper iodide (0.078 g) at room temperature, followed by stirring at 170°C for 4 hours. The reaction mixture was left to be cooled and then added with ethyl acetate, and the insoluble materials were separated by filtration through Celite. The filtrate was washed with 1 M hydrochloric acid, water, and saturated saline in this order, dried over anhydrous magnesium sulfate, and concentrated under reduced pressure. To the residue were added methanol (25 mL) and concentrated sulfuric acid (1.0 mL), followed by stirring at 100°C for 3 hours. Then, a saturated sodium bicarbonate solution (pH about 8) was added thereto under ice-cooling, and the precipitated solid was collected by filtration, washed with water, purified by silica gel column chromatography (hexane-ethyl acetate)=9:1) to obtain methyl 5-chloro-2-(3,4-difluorophenoxy)nicotinate (1.1 g).
Production Example 2 To a mixture of methyl 5-chloro-2-(3,4-difluorophenoxy)nicotinate (1.0 g), methanol (10 mL), and THF (10 mL) was added a 1 M aqueous sodium hydroxide solution (10 mL) at room temperature, followed by stirring for 11 hours. Then, 1 M hydrochloric acid (10 mL) was added thereto under ice-cooling, and the precipitated solid was collected by filtration and washed with water to obtain 5-chloro-2-(3,4-difluorophenoxy)nicotinic acid (0.94 g).
Production Example 3
To a mixture of 4-[(tert-butoxycarbonyl)(methyl)amino)benzoate (2.7 g), DMF (27 mL), and potassium carbonate (2.2 g) was added allyl bromide (1.1 mL) at room temperature, followed by stirring for 15 hours. Then, the organic layer was separated by the addition of water and ethyl acetate, washed with a saturated sodium bicarbonate solution, water, and saturated saline in this order, dried over anhydrous magnesium sulfate, and then concentrated under reduced pressure. To the residue were added dioxane (10 mL) and 4 M hydrochloric acid-dioxane solution (30 mL), followed by stirring for 30 hours. The reaction mixture was concentrated under reduced pressure, to the residue was added diethyl ether, and the precipitated solid was collected by filtration and washed with diethyl ether to obtain allyl 4-(methylamino)benzoate hydrochloride (2.09 g).

Production Example 4 To a mixture of allyl 4-(methylamino) benzoate hydrochloride (2.09 g), concentrated hydrochloric acid (10 mL), and water (10 mL) was added dropwise an aqueous solution (10 mL) of sodium nitrite (0.67 g) under ice-cooling, followed by stirring at the same temperature for 3 hours. The precipitated solid was collected by filtration and washed with 1 M hydrochloric acid and water. To the solid were added acetic acid (10 mL), water (5.0 mL), and zinc (2.7 g), followed by stirring at room temperature for 1.5 hours. The insoluble materials were separated by filtration through Celite, and the filtrate was concentrated under reduced pressure. To the residue were added ethyl acetate and a saturated sodium bicarbonate solution, the insoluble materials were separated by filtration, and then the organic layer was separated. The organic layer was washed with a saturated sodium bicarbonate solution, water, and saturated saline in this order, dried over anhydrous magnesium sulfate, and then filtered. The filtrate was concentrated under reduced pressure, and the residue was purified by silica gel column chromatography (hexane-ethyl acetate=3:1). To the obtained oily substance were added dioxane and a 4 M hydrochloric acid-dioxane solution, and the precipitated solid was collected by filtration. The solid was washed with diethyl ether to obtain allyl 4-(1-methylhydrazino) benzoate hydrochloride (1.3 g).
Production Example 5
To a mixture of methyl 5-oxo-5,6,7,8-tetrahydronaphthalene-2-carboxylate (0.93 g) and methanol (20 mL) were added hydroxylamine hydrochloride (0.35 g) and sodium acetate (0.41 g) at room temperature, followed by stirring for 11 hours. To the reaction mixture was added water, and the precipitated solid was collected by filtration and washed with water to obtain methyl 5-(hydroxyimino)-5,6,7,8-tetrahydronaphthalene-2-carboxylate (0.97 g).
Production Example 6
To a mixture of methyl 5-(hydroxyimino)-5,6,7,8-tetrahydronaphthalene-2-carboxylate (0.96 g) and methanol (20 mL) was added Raney nickel (1.0 mL), followed by stirring at room temperature under 3 atm for 24 hours under a hydrogen atmosphere. The insoluble materials were separated by filtration through Celite, and the filtrate was concentrated under reduced pressure. To the residue were added methanol and diethyl ether, added by 4 M hydrochloric acid-dioxane solution (2.0 mL) under ice-cooling and the precipitated solid was collected by filtration, and washed with diethyl ether to obtain methyl 5-amino-5,6,7,8-tetrahydronaphthalene-2-carboxylate hydrochloride (0.91 g).

Production Example 7
To a mixture of 3-[4-(tert-butoxycarbonylaminomethyl)phenyl]-1,2,4-oxadiazol-5(4H)-one (1.64 g) and ethyl acetate (4.9 mL) was added 4 M hydrochloric acid-ethyl acetate solution (21.1 mL), followed by stirring at room temperature for 4 hours. The reaction mixture was concentrated under reduced pressure, and the residue was washed with hexane (15 mL) to obtain 3-[4-(aminomethyl)phenyl]-1,2,4-oxadiazol-5(4H)-one hydrochloride (1.24 g) as a white solid.
Production Example 8
To a suspension oftert-butyl [(1S)-1-(4-cyanophenyl)ethyl]carbamate (2.44 g) and hydroxylamine hydrochloride (2.07 g) in ethanol (36.6 mL) was added triethylamine (4.14 mL) at room temperature, followed by stirring at 80°C overnight. Then, the mixture was left to be cooled to room temperature and water (100 mL) was added thereto, followed by extraction with ethyl acetate (120 mL). The organic layer was washed with a saturated sodium bicarbonate solution and saturated saline in this order, dried over magnesium sulfate, and then concentrated under reduced pressure to obtain tert-butyl [(1S)-1-{4-[amino(hydroxyimino)methyl]phenyl}ethyl]carbamate (2.44 g) as a white solid.
Production Example 9
A mixture of tert-butyl [(1S)-1-{4-[amino(hydroxyimino)methyl]phenyl}ethyl]carbamate (2.43 g), pyridine (1.06 mL), and DMF (36.5 mL) was ice-cooled, and 2-ethylhexyl chlorocarbonate (1.69 mL) was slowly added, followed by stirring at the same temperature for 1 hour. To the reaction mixture was added water (80 mL), followed by extraction with ethyl acetate (100 mL). The organic layer was washed with saturated saline, dried over magnesium sulfate, and concentrated under reduced pressure. The residue was washed with hexane (50 mL) to obtain tert-butyl [(1S)-1-(4-{amino[({[(2-ethylhexyl)oxy]carbonyl}oxy)imino]methyl}phenyl)ethyl]carbamate (3.5 g) as a white solid.

Production Example 10
A mixture of tert-butyl[(1S)-1-(4-{amino[({[(2-ethylhexyl)oxy]carbonyl}oxy)imino]methyl}phenyl)ethyl]carbamate (3.5 g) and xylene (35 mL) was stirred at an outer temperature of 140°C for 1 hour. After leaving it to be cooled to room temperature, the mixture was stirred under ice-cooling for 30 minutes.
The precipitated solid was collected by filtration and washed with diethyl ether (10 mL) to obtain tert-butyl {(1S)-1-[4-(5-oxo-4,5-dihydro-1,2,4-oxadiazol-3-yl)phenyl]ethyl}carbamate (2.2 g) as a white solid.
Production Example 11
To a mixture of methyl 2-amino-3-hydroxybenzoate (2.5 g), potassium carbonate (4.6 g), dichloroethane (20 mL), and water (20 mL) was added chloroacetyl chloride (1.3 mL), followed by stirring at 70°C overnight. The reaction mixture was left to be cooled, followed by addition of chloroform (40 mL) and water (40 mL), and the separated organic layer was washed with 1 M hydrochloric acid, a saturated sodium bicarbonate solution, and saturated saline in this order, dried over magnesium sulfate, and then concentrated under reduced pressure. The residue was recrystallized with ethyl acetate (2 mL) to obtain methyl 3-oxo-3,4-dihydro-2H-1,4-benzoxazine-5-carboxylate (746 mg) as a pale red crystal.
Production Example 12
A mixture of methyl 3-oxo-3,4-dihydro-2H-1,4-benzoxazine-5-carboxylate (735 mg) and DMF (11 mL) was ice-cooled and added with sodium hydride (156 mg), followed by stirring for 30 minutes. Then, 4-chlorobenzylbromide (765 mg) was added thereto, followed by stirring at the same temperature for 30 minutes and at room temperature for 2 hours. To the reaction mixture were added water (40 mL) and ethyl acetate (40 mL), and the separated organic layer was washed with 1 M hydrochloric acid, a saturated sodium bicarbonate solution, and saturated saline in this order, dried over magnesium sulfate, and then concentrated under reduced pressure. The residue was purified by silica gel column chromatography (hexane-ethyl acetate=3:1) to obtain methyl 4-(4-chlorobenzyl)-3-oxo-3,4-dihydro-2H-1,4-benzoxazine-5-carboxylate (1.07 g) as a colorless syrup.

Production Example 13
To a mixture of 5-chloro-1H-indole-7-carboxylic acid (500 mg), triphenyl phosphine (1.01 g), ethanol (235 mg) and toluene (20 mL) was added dropwise diethyl azodicarboxylate (2.2 M toluene solution, 1.74 mL) at room temperature, followed by stirring for 2 hours. The reaction mixture was concentrated under reduced pressure, and the obtained residue was purified by silica gel column chromatography (hexane-ethyl acetate) to obtain ethyl 5-chloro-1H-indole-7-carboxylate (550 mg) as a white solid.
Production Example 14
A mixture of methyl 2-amino-3-hydroxybenzoate (2 g) and methylene chloride (40 mL) was ice-cooled, and added triethylamine (3.34 mL) and 4-chlorophenylacetylchloride (1.93 mL) in this order, followed by stirring at the same temperature for 2 hours and at room temperature overnight. To the reaction mixture were added water (50 mL) and chloroform (50 mL), and the separated organic layer was washed with 1 M hydrochloric acid, a saturated sodium bicarbonate solution, and saturated saline in this order, dried over magnesium sulfate, and then concentrated under reduced pressure. The residue was dissolved in toluene (40 mL), and p-toluene sulfonic acid (2.47 g) was added thereto, followed by heating under reflux for 4 hours. After leaving it to be cooled, dilution with ethyl acetate (80 mL) was carried out, followed by washing with 1 M hydrochloric acid, a saturated sodium bicarbonate solution, and saturated saline in this order. The precipitated solid was removed by filtration, and then the filtrate was dried over magnesium sulfate, and concentrated under reduced pressure. The residue was purified by silica gel column chromatography (hexane-ethyl acetate=3:1) to obtain methyl 2-(4-chlorobenzyl)-1,3-benzoxazole-4-carboxylate (850 mg) as a pale yellow syrup.
Production Example 15
To a mixture of methyl 2-chloro-3-nitrobenzoate (1.50 g) and THF (15 mL) were added triethylamine (1.94 mL) and p-chlorobenzylamine (934 µL) at room temperature, followed by stirring at 60°C overnight. The reaction liquid was left to be cooled and then concentrated under reduced pressure, and to the residue was added water, followed by extraction with chloroform. The organic layer was dried over anhydrous sodium sulfate and concentrated under reduced pressure. The residue was recrystallized from ethanol to obtain methyl 2-[(4-chlorobenzyl)amino]-3-nitrobenzoate (1.63 g) as a yellow crystal.

Production Example 16
A mixture of methyl 2-[(4-chlorobenzyl)amino]-3-nitrobenzoate (1.43 g), iron (1.24 g), and acetic acid (14 mL) was stirred at 60°C for 3 hours. After leaving it to be cooled, the reaction solution was diluted with ethanol, and the insoluble materials were removed by filtration through Celite. The filtrate was concentrated under reduced pressure, and a solution obtained by adding ethyl acetate to the residue was washed twice with a saturated sodium bicarbonate solution, and dried over anhydrous sodium sulfate. Further, the residue obtained by concentration under reduced pressure was purified by silica gel column chromatography (hexane-ethyl acetate) to obtain methyl 3-amino-2-[(4-chlorobenzyl)ammo]benzoate (1.13 g) as a yellow oily substance.
Production Example 17
To a mixture of methyl 3-amino-2-[(4-chlorobenzyl)amino]benzoate (200 mg) and acetic acid (4 mL) was added sodium nitrite (57 mg) at room temperature, followed by stirring overnight. To the reaction solution was added toluene, followed by concentration under reduced pressure. The obtained residue was purified by silica gel column chromatography (hexane-ethyl acetate) to obtain methyl 1-(4-chlorobenzyl)-1H-benzotriazole-7-carboxylate (199 mg) as a white solid.
Production Example 18
To a mixture of 4-hydrazinobenzoic acid (1.0 g) and tert-butyl acetate (15 mL) was added borotrifluoride-diethyl ether complex (1.8 mL) at room temperature, followed by stirring at room temperature for 3 hours. The reaction mixture was added to a 1 M aqueous sodium hydroxide solution (50 mL) to separate the organic layer, followed by washing with water and saturated saline. After dried over anhydrous magnesium sulfate and concentrated under reduced pressure, the residue was purified by silica gel column chromatography (hexane:ethyl acetate=3:1→+2:1) to obtain tert-butyl 4-hydrazinobenzoate (0.22 g).

Production Example 19
A mixture of tert-butyl 7-({(1S)-1-[4-(methoxycarbonyl)phenyl]ethyl}carbamoyl)indoline-1-carboxylate (972 mg), methanol (4 mL), and a 4 M hydrochloric acid-dioxane solution (8 mL) was stirred at room temperature for 6 hours. The reaction mixture was concentrated under reduced pressure to obtain methyl 4- {(1S)-1-[(2,3-dihydro-1H-indol-7-ylcarbonyl)amino]ethyl}benzoate hydrochloride (989 mg).
Production Example 20
To a mixture of methyl 1-(4-chlorobenzyl)-1H-indole-7-carboxylic acid (200 mg) and DMF (2 mL) was added dropwise phosphorous oxychloride (110 µL) under ice-cooling. The mixture was stirred at room temperature for 20 minutes and at 60°C for 12 hours. After leaving it to be cooled to room temperature, the reaction mixture was put into water, added with a saturated sodium bicarbonate for neutralization, and then extracted with a mixed solution of ethyl acetate and diethyl ether. The organic layer was washed with water and saturated saline in this order, dried over anhydrous sodium sulfate, and concentrated under reduced pressure. To the obtained residue were added THF (2 mL) and a BH₃/THF complex (1.0 M THF solution, 1 mL) at room temperature, followed by stirring at 60°C for 2 hours. To the reaction mixture was slowly added methanol under ice-cooling, and concentrated under reduced pressure, and the residue was purified by silica gel column chromatography (hexane-ethyl acetate) to obtain methyl 1-(4-chlorobenzyl)-3-methyl-1H-indole-7-carboxylate (66 mg) as a white solid.
Production Example 21
To a mixture of 1-aminoindane-5-carbonitrile hydrochloride (403 mg) and water (4.0 mL) was added concentrated sulfuric acid (0.40 mL), followed by stirring at 100°C overnight. After leaving it to be cooled, the mixture was concentrated under reduced pressure, methanol (10 mL) was added thereto, followed by stirring at 80°C for 3 days. Next, water and potassium carbonate were added thereto, followed by extraction with ethyl acetate. The organic layer was washed with saturated saline, dried over anhydrous sodium sulfate, and then concentrated under reduced pressure to obtain methyl 1-aminoindane-5-carboxylate (152 mg) as a white solid.

Production Example 22
To a mixture of methyl 1H-indole-7-carboxylate (2.00 g) and tert-butanol (100 mL) was slowly added N-bromosuccinimide (1.10 g) at room temperature, followed by stirring for 1 hour. Additionally, N-bromosuccinimide (2.20 g) was added thereto, followed by stirring at room temperature for 2 hours. The reaction mixture was concentrated under reduced pressure, and then to the residue was added a saturated sodium bicarbonate solution, followed by extraction with ethyl acetate. The organic layer was dried over anhydrous sodium sulfate and then concentrated under reduced pressure, and the obtained residue was purified by silica gel column chromatography (hexane-ethyl acetate) to obtain methyl 3-bromo-1H-indole-7-carboxylate (490 mg) as a white solid.
Production Example 23
To 1-(tert-butoxycarbonyl)indoline-6-carboxylic acid (4.58 g) was added DMF (46 mL), and potassium carbonate (3.61 g) and allyl bromide (2.53 g) were added thereto under ice-cooling, followed by stirring at room temperature for 2 days. To the reaction mixture were added water (50 mL) and 1 M aqueous sodium hydroxide solution (50 mL) under ice-cooling, followed by extraction with ethyl acetate. The organic layer was washed with saturated saline, dried over sodium sulfate, and concentrated under reduced pressure. The obtained residue was purified by silica gel column chromatography (hexane-ethyl acetate=20/1 to 10/1) to obtain 6-allyl 1-tert-butyl indoline-1,6-dicarboxylate (4.95 g) as a colorless oily substance.
Production Example 24 To a mixture of ethyl 5-(1-hydroxy ethyl)thiophene-2-carboxylate (1,01 g), diphenylphosphorylazide (1.67 g), and toluene (10 mL) was added 1,8-diazabicyclo[5.4.0]undec-7-ene (905 µL) under ice-cooling, followed by stirring under ice-cooling for 30 minutes and stirring at room temperature for 15 hours. The reaction solution was washed with water and 1 M hydrochloric acid in this order, and dried over anhydrous sodium sulfate. Then, the residue obtained by concentration under reduced pressure was purified by silica gel column chromatography (hexane-ethyl acetate) to obtain ethyl 5-(1-azidoethyl)thiophene-2-carboxylate (1.03 g) as a colorless oily substance.

Preparative Example 25
To a mixture of ethyl 5-(1-azidoethyl)thiophene-2-carboxylate (1.03 g), THF (20 mL), and water (4 mL) was added triphenyl phosphine (2.35 g) at room temperature, followed by stirring at 60°C for 3 hours. After leaving it to be cooled, the mixture was concentrated under reduced pressure and azeotroped with toluene. The obtained residue was purified by silica gel column chromatography (chloroform-methanol), 4 M hydrochloric acid-ethyl acetate solution (1.5 mL) was added thereto, followed by stirring for 3 minutes, and the mixture was concentrated again under reduced pressure.
Diisopropyl ether was added thereto, and the precipitated white solid was collected by filtration to obtain ethyl 5-(1-aminoethyl)thiophene-2-carboxylate hydrochloride (979 mg) as a white solid.
Preparative Example 26
To a mixture of methyl 1H-indole-7-carboxylate (100 mg) and DMF, (1 mL) was added potassium tert-butoxide (75 mg) at room temperature, followed by stirring for 5 minutes, and then 4-methoxybenzyl chloride (91 µL) was added thereto, followed by stirring at room temperature for 2 hours. Then, water and ethyl acetate were added thereto, and the separated organic layer was washed with water and saturated saline in this order, dried over anhydrous sodium sulfate, and then concentrated under reduced pressure to obtain a residue (216 mg). To the obtained residue were added THF (10 mL), methanol (5 mL), and a 1 M aqueous sodium hydroxide solution (2.5 mL), followed by stirring at 60°C overnight. After leaving it to be cooled, to the residue obtained by concentration under reduced pressure was added ethyl acetate, followed by extraction with water. To the aqueous layer were added ethyl acetate and 1 M hydrochloric acid (2.5 mL), and the separated organic layer was dried over anhydrous sodium sulfate and then concentrated under reduced pressure to obtain 1-(4-methoxybenzyl)-1H-indole-7-carboxylic acid (157 mg) as a pale yellow solid.
Production Example 27
A mixture of 7-bromo-1-(4-chlorobenzyl)-2-methyl-1H-indole (360 mg) and THF (5 mL) was cooled to -78°C under an argon atmosphere, and n-butyllithium (1.58 M hexane solution, 749 µL) was added dropwise thereto. After stirring at the same temperature for 30 minutes, dry ice (1.0 g) was added thereto, followed by elevating the temperature slowly to room temperature and stirring at 30 minutes. Then, 1 M hydrochloric acid was added thereto (acidic pH), followed by extraction with ethyl acetate. The organic layer was washed with saturated saline, dried over anhydrous sodium sulfate, and then concentrated under reduced pressure to obtain 1-(4-chlorobenzyl)-2-methyl-1H-indole-7-carboxylic acid (310 mg) as a pale yellow solid.

Production Example 28
To a mixture of methyl 3-amino-2-hydroxybenzoate (700 mg) and THF (21 mL) was added 4-chlorophenylisothiocyanate (717 mg), followed by stirring at room temperature overnight. To the reaction mixture were added copper iodide (0.87 g) and triethylamine (641 µL), followed by stirring at 60°C overnight. The reaction mixture was concentrated under reduced pressure and dissolved in methanol, the resulting black precipitate was filtered through Celite, and the filtrate was concentrated under reduced pressure. To the residue was again added ethyl acetate (20 mL), the insoluble materials were removed by filtration, and the filtrate was concentrated under reduced pressure. The residue was purified by silica gel column chromatography (hexane:ethyl acetate=2:1), and then washed with hexane-ethyl acetate (10:1, 11 mL) to obtain methyl 2-[(4-chlorophenyl)amino]-1,3-benzoxazole-7-carboxylate (270 mg) as a pale yellow solid.
Production Example 29
To a mixture of 2-(5-chloro-1H-indol-1-yl)benzonitrile (753 mg) and ethyleneglycol (10.0 mL) was added a 1 M aqueous sodium hydroxide solution (15.0 mL), followed by stirring at 180°C for 6 hours. After leaving it to be cooled to room temperature, 1 M hydrochloric acid (15.0 mL) was added thereto for neutralization, followed by extraction with ethyl acetate. The organic layer was washed with water and saturated saline, dried over anhydrous sodium sulfate, and concentrated under reduced pressure to obtain 2-(5-chloro-1H-indol-1-yl)benzoate (844 mg).
Production Example 30
A oxygen monoxide gas was passed through a mixture of tert-butyl [(1R)-1-(4-bromo phenyl)ethyl]carbamate (1.3 g), 1,3-propanediyl bis(diphenylphosphine) (0.18 g), palladium diacetate (0.10 g), DMF (13 mL), methanol (20 mL), and triethylamine (1.9 mL) at room temperature, followed by stirring at 80°C for 17.5 hours. After leaving it to be cooled, water was added thereto, followed by extraction with diethyl ether. The organic layer was washed with water, dried over anhydrous magnesium sulfate, and then concentrated under reduced pressure, and the residue was purified by silica gel column chromatography (hexane:ethyl acetate=7:1) to obtain methyl 4-{(1R)-1-[(tert-butoxycarbonyl)amino]ethyl}benzoate (1.1 g).

Production Example 31
A mixture of methyl indole-7-carboxylate (6.0 g) and DMF (60 mL) was ice-cooled, and potassium tert-butoxide (5.38 g) was added thereto, followed by stirring at the same temperature for 30 minutes. Then, 4-chlorobenzylbromide (7.39 g) was added thereto, followed by stirring at room temperature for 3 hours. The reaction mixture was ice-cooled, and water (60 mL) was added thereto, followed by extraction with ethyl acetate (80 mL). The organic layer was washed with 1 M hydrochloric acid, a saturated sodium bicarbonate solution, and saturated saline in this order, dried over magnesium sulfate, and then concentrated under reduced pressure. The residue was left to stand for 3 days for solidification, and washed with hexane-ethyl acetate (8:1, 18 mL) to obtain methyl 1-(4-chlorobenzyl)-1H-indole-7-carboxylate (4.94 g) as a pale yellow solid.
Production Example 32
To a mixture of 1-methyl-2H-thieno[3,2-d][1,3]oxazine-2,4 (1H)-dione (0.16 g) and ethanol (5.0 mL) were added triethylamine (0.18 mL) and methyl 4-[(1S)-1-aminoethyl]benzoate hydrochloride (0.22 g), followed by heating under reflux for 13 hours. After leaving it to be cooled to room temperature, a 10% aqueous citric acid solution was added thereto under ice-cooling, and the precipitated solid was collected by filtration and washed with water to obtain methyl 4-[(1S)-1-({[3-(methylamino)-2-thienyl]carbonyl}amino)ethyl]benzoate (0.23 g).

The compounds of Production Examples were prepared by the same production processes as for the compounds of Production Examples 1 to 32 as shown in Tables below, using each of the corresponding starting materials. The structures, the production processes, and the physicochemical data of the compounds of Production Examples are shown in Tables 2 to 11.

Example 1 (Production Process A)
To a mixture of 5-chloro-2-(3,4-difluorophenoxy)nicotinic acid (0.30 g), allyl 4-(1-methylhydrazino)benzoate hydrochloride (0.28 g), and DMF (6.0 mL) were added HOBt (0.16 g) and WSC (0.26 mL) at room temperature, followed by stirring for 3 days. Then, water was added thereto, followed by extraction with ethyl acetate, and the organic layer was washed with 10% aqueous citric acid solution, a saturated sodium bicarbonate solution, water, and saturated saline in this order. Additionally, it was dried over anhydrous magnesium sulfate and then concentrated under reduced pressure to obtain allyl 4-(2-{[5-chloro-2-(3,4-difluorophenoxy)pyridin-3-yl]carbonyl}-1-methylhydrazino)benzoate (0.55 g).
Example 2 (Production Process B)
To a mixture of allyl 4-(2-{[5-chloro-2-(3,4-difluorophenoxy)pyridin-3-yl]carbonyl}-1-methylhydrazino)benzoate (0.54 g) and THF (5.0 mL) were added tetrakis(triphenylphosphino)palladium (IV) (66 mg) and morpholine (0.50 mL) under ice-cooling, followed by stirring under ice-cooling for 0.5 hour, at room temperature for 3 hours, and then at 50°C for 24 hours. Morpholine (1.0 mL) and THF (10 mL) were further added thereto, followed by stirring at 50°C for 13 hours. After leaving it to be cooled, tetrakis(triphenylphosphino)palladium (IV) (0.13 g) was added thereto, followed by further stirring at 50°C for 8 hours. The reaction solution was left to be cooled, and a 10% aqueous citric acid solution was added thereto, followed by extraction with ethyl acetate. The organic layer was washed with water and saturated saline, dried over anhydrous magnesium sulfate, and then concentrated under reduced pressure. The residue was purified by silica gel column chromatography (hexane:THF=3:1+1% formic acid), and the obtained solid was washed with diethyl ether to obtain 4-(2-{[5-chloro-2-(3,4-difluorophenoxy)pyridin-3-yl]carbonyl}-1-methylhydrazino)benzoate (0.28 g).
Example 3 (Production Process C)
To a mixture of tert-butyl 4-(2-{[5-chloro-2-(3,4-difluorophenoxy)pyridin-3-yl]carbonyl}hydrazino)benzoate (0.13 g) and methylene chloride (2.0 mL) was added trifluoroacetic acid (2.0 mL) at room temperature, followed by stirring for 0.5 hour. The reaction mixture was concentrated under reduced pressure, to the residue was added water, and the precipitated solid was collected by filtration. The obtained solid was washed with water and recrystallized from ethyl acetate-hexane to obtain 4-(2-{[5-chloro-2-(3,4-difluorophenoxy)pyridin-3-yl]carbonyl}hydrazino)benzoate (0.050 g).

Example 4 (Production Process D)
To a mixture of methyl 1-{[2-(4-chlorobenzyl)benzoyl]amino}indoline-5-carboxylate (308 mg), methanol (2 mL), and THF (2 mL) was added 1 M aqueous sodium hydroxide solution (2 mL), followed by stirring at 60°C overnight. After leaving it to be cooled to room temperature, a 10% aqueous citric acid solution (5 mL) was added thereto, followed by stirring. The precipitated solid was collected by filtration and washed with water, and the obtained solid was recrystallized with 60% aqueous ethanol solution to obtain a white solid. It was further purified by preparative high performance liquid chromatography (ODS; acetonitrile-0.1% aqueous trifluoroacetic acid solution) to obtain 1-{[2-(4-chlorobenzyl)benzoyl]amino}-1H-mdole-5-carboxylic acid (5 mg).
Example 5 (Production Process E)
To a mixture of 1-(4-chlorobenzyl)-1H-mdole-7-carboxylic acid (0.20 g) and THF (2.0 mL) were added oxalyl chloride (0.10 mL) and one droplet of DMF under ice-cooling, followed by stirring at the same temperature for 0.5 hour. The reaction mixture was concentrated under reduced pressure, and the residue was azeotroped with toluene. To the obtained residue was added pyridine (4.0 mL), and methyl 4-(1-isopropylhydrazino)benzoate hydrochloride (0.19 g) was added thereto under ice-cooling, followed by stirring at room temperature for 3 hours. To the reaction mixture was added water under ice-cooling, followed by extraction with ethyl acetate. The organic layer was washed with a 10% aqueous citric acid solution, a saturated sodium bicarbonate solution, water, and saturated saline, dried over anhydrous magnesium sulfate, and concentrated under reduced pressure. The residue was purified by silica gel column chromatography (ethyl acetate-hexane=0→30%) to obtain methyl 4-(2-{[1-(4-chlorobenzyl)-1H-indol-7-yl]carbonyl}-1-isopropylhydrazino}benzoate (0.22 g).
Example 6 (Production Process F)
To allyl indoline-6-carboxylate hydrochloride (1.5 g) were added concentrated hydrochloric acid (1.5 mL) and water (3 mL), and a solution of sodium nitrite (0.47 g) and water (1.5 mL) were slowly added dropwise thereto under ice-cooling. Next, water (15 mL) and concentrated hydrochloric acid (15 mL) were added thereto, followed by stirring for 30 minutes, and then acetic acid (20 mL) and zinc (2.5 g) were further added thereto, followed by stirring at room temperature overnight. The reaction mixture liquid was filtered through Celite, and the filtrate was washed with ethyl acetate. The aqueous layer was neutralized with a saturated sodium bicarbonate solution, and ethyl acetate was added thereto. The separated organic layer was washed with saturated saline, dried over sodium sulfate, and then concentrated under reduced pressure. The obtained residue was purified by silica gel column chromatography (hexane-ethyl acetate=9/1 to 1/1) to obtain allyl 1-aminoindoline-6-carboxylate (248 mg) as a yellow oily substance.
To a mixture of allyl 1-aminoindoline-6-carboxylate (238 mg), 1-(4-chlorobenzyl)-1H-indole-7-carboxylie acid (260 mg), and DMF (4 mL) were added HATU (415 mg) and DIPEA (190 µL) under ice-cooling, followed by stirring at 60°C for 22 hours. After leaving it to be cooled, a 10% aqueous citric acid solution was added thereto to adjust the pH to about 3, followed by extraction with ethyl acetate, and the organic layer obtained was washed with a saturated sodium bicarbonate solution and saturated saline in this order. It was further dried over sodium sulfate and then concentrated under reduced pressure, and the obtained residue was purified by silica gel column chromatography (hexane-ethyl acetate=95/5-70/30) to obtain allyl 1-({[1(4-chlorobenzyl)-1H-indol-7-yl]carbonyl}amino)indoline-6-carboxylate (260 mg).

Example 9 (Production Process G)
To a mixture of methyl 5-({[5-chloro-2-(3,4-difluorophenoxy)pyridin-3-yl]carbonyl}amino)-5,6,7,8-tetrahydronaphthalene-2-carboxylate (0.30 g), methanol (3.0 mL), and THF (3.0 mL) was added a 1 M aqueous sodium hydroxide solution (3.0 mL) at room temperature, followed by stirring for 8 hours. Then, 1 M hydrochloric acid (3.0 mL) was added thereto, and the precipitated crystal was collected by filtration and washed with water. The obtained crystal was recrystallized from THF to obtain 5-({[5-chloro-2-(3,4-difluorophenoxy)pyridin-3-yl]carbonyl}amino)-5,6,7,8-tetrahydronaphthalene-2-carboxylic acid (0.043 g).
Example 10 (Production Process H)
To a mixture of methyl 4-{(1S)-1-[(2,3-dihydro-1H-indol-7-ylcarbonyl)amino]ethyl}benzoate hydrochloride (140 mg) and potassium carbonate (134 mg) were added DMF (3 mL) and p-chlorobenzyl bromide (100 mg) in this order. The reaction mixture was stirred at room temperature for 1 hour, and then stirred at 90°C for 2 hours. The reaction mixture was left to be cooled to room temperature, and then water was added thereto, followed by extraction with ethyl acetate. This organic layer was dried over anhydrous sodium sulfate and then concentrated under reduced pressure, and the obtained residue was purified by silica gel column chromatography (hexane-ethyl acetate) to obtain methyl 4-[(1S)-1-({[1-(4-chlorobenzyl)-2,3-dihydro-1H-indol-7-yl]carbonyl}amino)ethyl]benzoate (135 mg) as a colorless amorphous substance.
Example 11 (Production Process I)
To a mixture of methyl 4-[(1S)-1-({[3-(methylamino)-2-thienyl]carbonyl}amino)ethyl]benzoate (0.22 g) and 1,3-dimethylimidazolidin-2-one (2.0 mL) were added potassium carbonate (150 mg) and 4-chlorobenzyl bromide (175 mg), followed by stirring at 50°C for 8 hours. To the mixture were further added potassium carbonate (75 mg) and 4-chlorobenzyl bromide (88 mg), followed by stirring at 50°C for 14 hours. Additionally, potassium carbonate (150 mg) and 4-chlorobenzyl bromide (175 mg) were added thereto, followed by stirring at 50°C for 2 days. To the reaction mixture was added a 10% aqueous citric acid solution under ice-cooling, followed by extraction with ethyl acetate. The organic layer was washed with a 10% aqueous citric acid solution, a saturated sodium bicarbonate solution, water, and saturated saline, dried over anhydrous magnesium sulfate, and then concentrated under reduced pressure. The residue was purified by silica gel column chromatography (THF-hexane=10%→30%) to obtain methyl 4-{(1S)-1-[({3-[(4-chlorobenzyl)(methyl)amino]-2-thienyl}carbonyl)amino]ethyl}benzoate (0.26 g).
Example 12 (Production Process J)
To a mixture of 1-(4-chlorobenzyl)-1H-indole-7-carboxylic acid (8.6 mg), methyl 6-aminohexanoate hydrochloride (6.4 mg), HOBt (4.1 mg), triethylamine (9.7 µL), and DMF (1.0 mL) was added PS-carbodiimide (100 mg) at room temperature, followed by stirring overnight. To the reaction solution were further added MP-carbonate (50 mg) and PS-isocyanate (50 mg), followed by stirring at room temperature for 4 hours, and the insoluble materials were filtered. The filtrate was concentrated under reduced pressure, the obtained residue was dissolved in a mixed solvent of ethanol (0.5 mL) and THF (0.5 mL), and a 1 M aqueous sodium hydroxide solution (0.5 mL) was added thereto at room temperature, followed by stirring overnight. The reaction solution was purified by solid phase extraction using OASIS (registered trademark) MAX manufactured by Waters (eluent, formic acid:methanol=1:49) to obtain 6-({[1-(4-chlorobenzyl)-1H-indol-7-yl]carbonyl}amino)hexanoic acid (1.0 mg).

The compounds of Examples as shown in Tables below were prepared in the same manner as the methods of Examples above, using each of the corresponding starting materials. The structures of the compounds of Examples 1 to 114 are shown in Tables 12 to 25, and the production processes and the physicochemical data of the compounds are shown in Tables 26 to 32.
Additionally, the following abbreviations are used in Production Examples, Examples, and Tables to be described later. Pre: Production Example number, Ex: Example number, Str: Structural formula, Syn: Production process (in Examples/ Production Examples above, representing the production process of Production Example number and Example having the same preparation manner. For example, the compound of Production Example 34 represents that as prepared in the same manner as for the compound of Production Example 5. Furthermore, the compounds prepared in Production Processes A to J of Examples each represent those as prepared in the same manner as for the compounds of Examples 1 to 6 and Examples 9 to 12), Dat: Physicochemical data (NMR1: δ (ppm) in 1H NMR in DMSO-d₆, NMR2: δ (ppm) in 1H NMR in CDCl₃, FAB+: FAB-MS (cation), FAB-: FAB-MS (anion), ESI+: ESI-MS (cation), ESI-: ESI-MS (anion), EI: EI-MS (cation), CI+: CI-MS (cation)), Me: Methyl, Et: ethyl, nPr: Normal propyl, nBu: Normal butyl, ¹Pr: Isopropyl, ^{t}Bu: tert-Butyl, Boc: tert-Butoxycarbonyl.

**[Table 2]**

| Pre | Syn | Str | Dat |
|---|---|---|---|
| 1 | 1 | 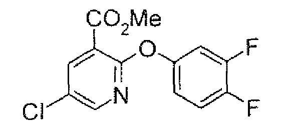 | ESI+: 300 |
| 2 | 2 | 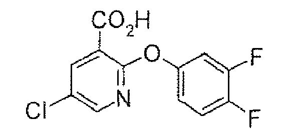 | ESI+: 286 |
| 3 | 3 | 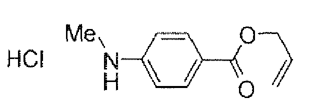 | NMR1: 7.73 (2H, d, J = 8.8 Hz), 6.58 (2H, d, J = 8.8 Hz), 6.07-5-96 (1H, m), 5.39-5.32 (1H, m), 5.26-5.21 (1H, m), 5.16-4.87 (1H, brs), 4.72-4.68 (2H, m), 2.72 (3H, s). |
| 4 | 4 | 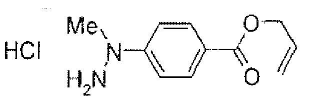 | ESI+: 248 (M+MeCN+H)+ |
| 5 | 5 | 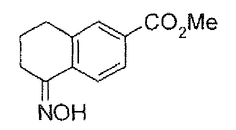 | ESI+: 220 |
| 6 | 6 | 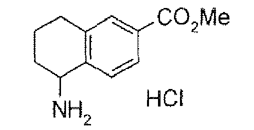 | NMR1: 8.72-8.58 (2H, brs), 7.83-7.7 (3H, m), 4.52-4.43 (1H, m), 3.85 (3H, s), 2.92-2.72 (2H, m), 2.16-2.04 (1 H, m), 2.00-1.87 (211, m), 1.81-1.69 (1H, m). |
| 7 | 7 | 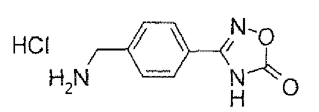 | ESI+: 192 |
| 8 | 8 | 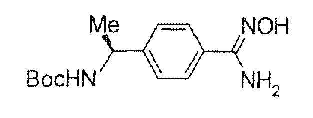 | ESI+: 280 |

**[Table 3]**

| | | | |
|---|---|---|---|
| 9 | 9 | 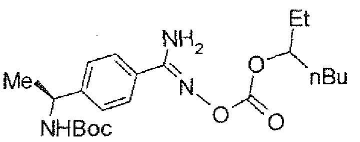 | ESI+: 436 |
| 10 | 10 | 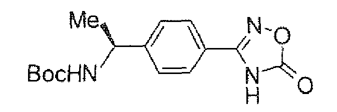 | ESI+: 306 |
| 11 | 11 | 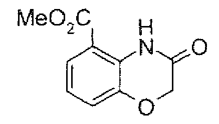 | ESI+: 208 |
| 12 | 12 | 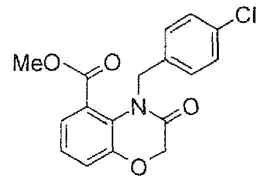 | ESI+: 332 |
| 13 | 13 | 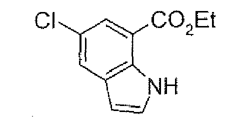 | ESI+: 224 |
| 14 | 14 | 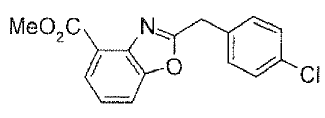 | ESI+: 302 |
| 15 | 15 | 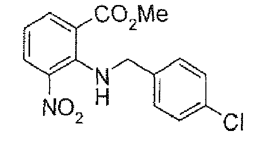 | FAB+: 321 |
| 16 | 16 | 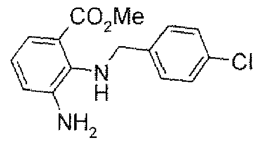 | ESI+: 291 |

**[Table 4]**

| | | | |
|---|---|---|---|
| 17 | 17 | 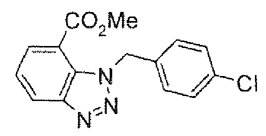 | ESI+: 302 |
| 18 | 18 | 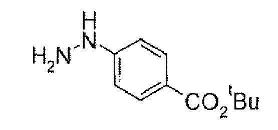 | ESI+: 209 |
| 19 | 19 | 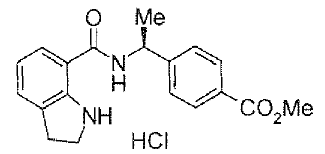 | ESI+: 325 |
| 20 | 20 | 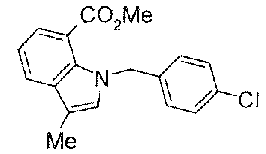 | ESI+: 314, EI: 313 |
| 21 | 21 | 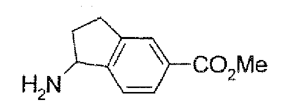 | EI: 191 |
| 22 | 22 | 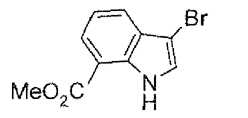 | EI: 253 |
| 23 | 23 | 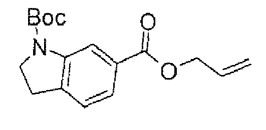 | EI: 303 |

**[Table 5]**

| | | | |
|---|---|---|---|
| 24 | 24 | 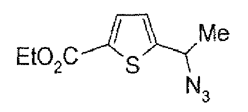 | CI+: 226 |
| 25 | 25 | 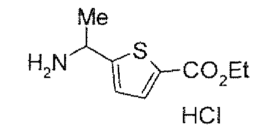 | FAB+: 200 |
| 26 | 26 | 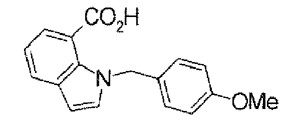 | ESI+: 282 |
| 27 | 27 | 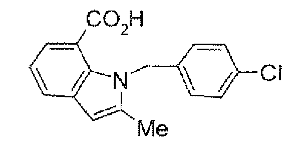 | ESI+: 300 |
| 28 | 28 | 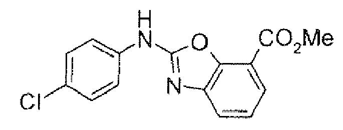 | ESI+: 303 |
| 29 | 29 | 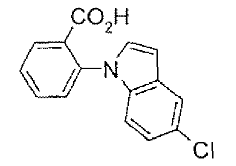 | ESI-: 270 |
| 30 | 30 | 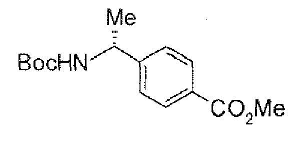 | ESI+: 280 |

**[Table 6]**

| | | | |
|---|---|---|---|
| 31 | 31 | 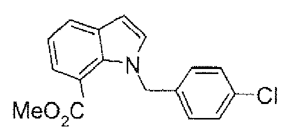 | ESI+: 300 |
| 32 | 32 | 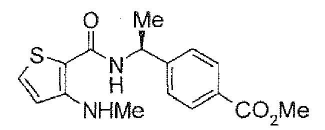 | ESI+: 319 |
| 33 | 31 | 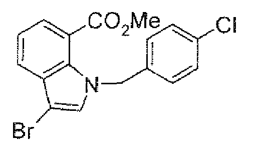 | EI: 377 |
| 34 | 5 | 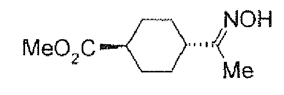 | NMR2: 8.42-8.31 (1H, brs), 3.68 (3H, s), 2.33-2.23 (1H, m), 2.19-2.02 (3H, m), 1.97-1.89 (2H, m), 1.86 (3H, s), 1.58-1,25 (4H, m) |
| 35 | 6 | 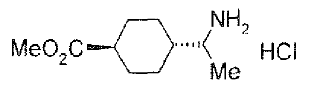 | ESI+: 186 |
| 36 | 7 | 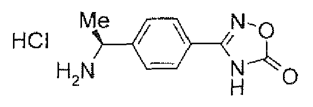 | ESI+: 206 |
| 37 | 2 | 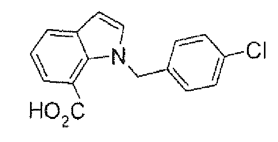 | ESI+: 286 |

**[Table 7]**

| | | | |
|---|---|---|---|
| 38 | 31 | 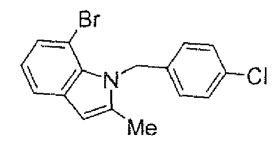 | NMR1: 7.51 (1H, dd, J = 0.9, 7.8 Hz), 7.36 (2H, d, J = 8.5 Hz), 7.23 (1H, dd, J = 0.9, 7.8 Hz), 6.92 (1H, t, J = 7.8 Hz), 6.84 (2H, d, J = 8.5 Hz), 6.43 (1H, d, J = 1.0 Hz), 5.8 (2H, s), 2.30 (3H, s). |
| 39 | 4 | 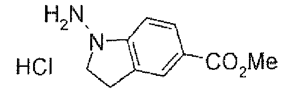 | EI: 192 |
| 40 | 2 | 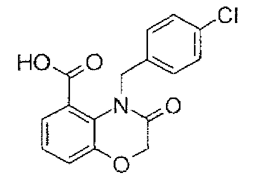 | ESI+: 359 (M+MeCN+H)+ |
| 41 | 7 | 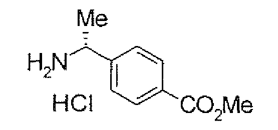 | ESI+: 221 (M+MeCN+H)+ |
| 42 | 19 | 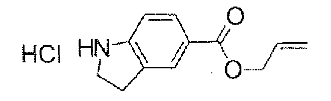 | ESI+: 204 |
| 43 | 4 | 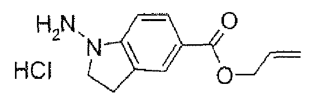 | EI: 218 |
| 44 | 31 | 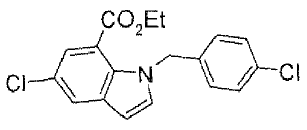 | ESI+: 348 |
| 45 | 2 | 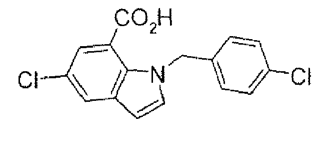 | ESI+: 320 |

**[Table 8]**

| | | | |
|---|---|---|---|
| 46 | 2 | 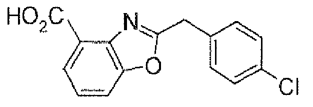 | ESI+: 288 |
| 47 | A | 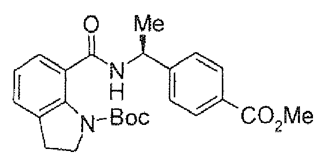 | ESI+: 425 |
| 48 | 2 | 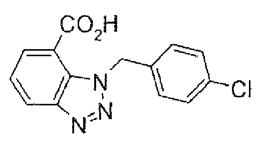 | ESI+: 288 |
| 49 | 2 | 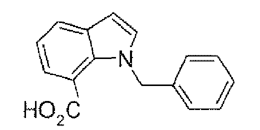 | ESI+: 252 |
| 50 | 2 | 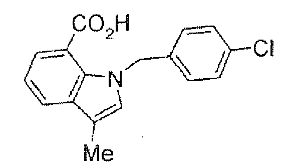 | ESI+: 300 |
| 51 | 2 | 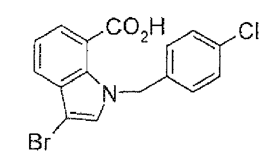 | FAB+: 364 |
| 52 | 4 | 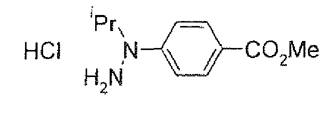 | ESI+: 209 |

**[Table 9]**

| | | | |
|---|---|---|---|
| 53 | 31 | 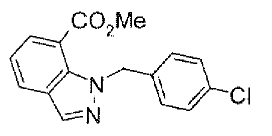 | NMR2: 8.16 (1H, s), 7.94 (1H, dd, J = 1.0, 7.8 Hz), 7.89 (1H, dd, J = 1.0, 7.3 Hz), 7.21-7.15 (3H, m), 6.93 (2H, d, J = 8.7 Hz), 5.98 (2H, s), 3.84 (3H, s); ESI+: 301 |
| 54 | 31 | 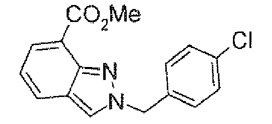 | NMR2: 8.10 (1H, dd, J = 1.0, 7.0 Hz), 7.90 (1H, s), 7.85 (1H, dd, J = 1.0, 8.3 Hz), 7.38-7.33 (2H, m), 7.30-7.25 (2H, m), 7.15 (1H, dd, J = 7.0, 8.3 Hz), 5.69 (2H, s), 4.03 (3H, s); ESI+: 301 |
| 55 | 31 | 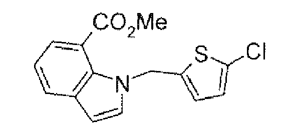 | ESI+: 306 |
| 56 | 19 | 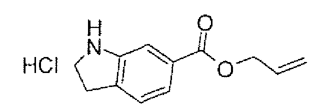 | ESI+: 204 |
| 57 | 2 | 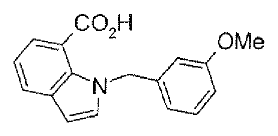 | ESI+: 282 |
| 58 | 2 | 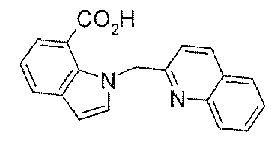 | ESI+: 303 |
| 59 | 2 | 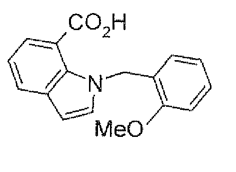 | ESI+: 282 |

**[Table 10]**

| | | | |
|---|---|---|---|
| 60 | 2 | 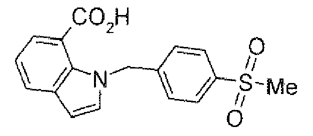 | ESI+: 330 |
| 61 | 23 | 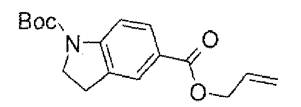 | EI: 303 |
| 62 | 14 | 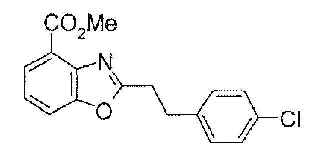 | ESI+: 316 |
| 63 | 2 | 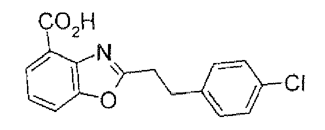 | ESI+: 302 |
| 64 | 2 | 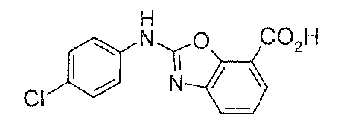 | ESI+: 289 |
| 65 | 2 | 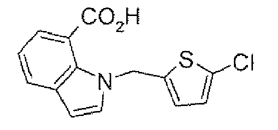 | ESI+: 292 |
| 66 | 2 | 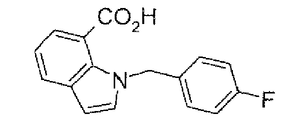 | ESI+: 270 |
| 67 | 2 | 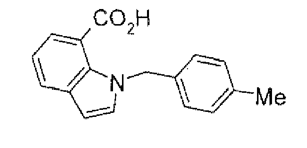 | ESI+: 266 |

**[Table 11]**

| | | | |
|---|---|---|---|
| 68 | 2 | 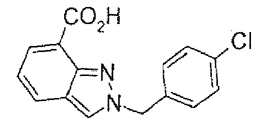 | ESI+: 287 |
| 69 | 2 | 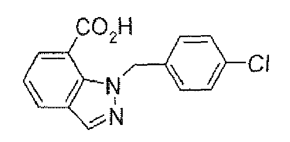 | ESI+: 287 |
| 70 | 31 | 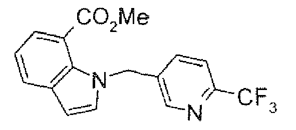 | ESI+: 335 |
| 71 | 31 | 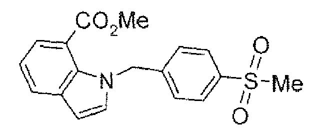 | ESI+: 344 |
| 72 | 31 | 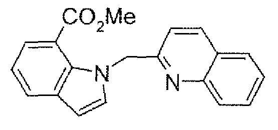 | ESI+: 317 |
| 73 | 2 | 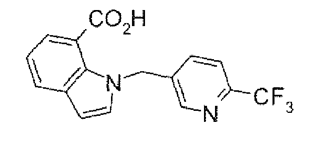 | ESI+: 321 |

**[Table 12]**

| Ex | Str | | Ex | Str |
|---|---|---|---|---|
| 1 | 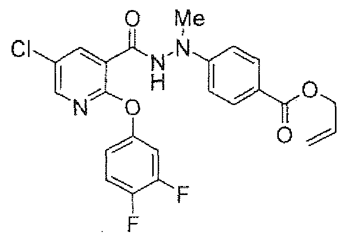 | | 2 | 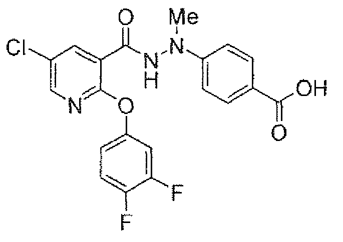 |
| 3 | 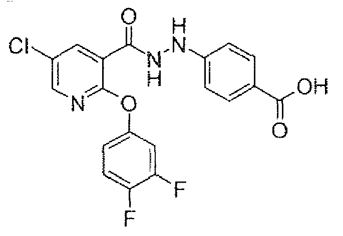 | | 4 | 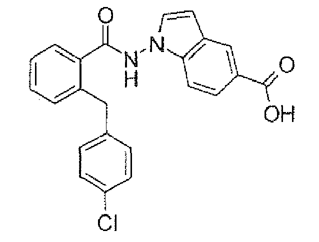 |
| 5 | 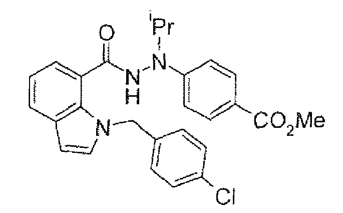 | | 6 | 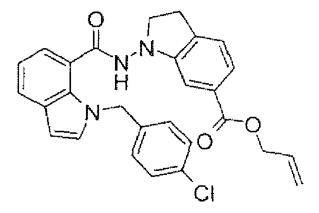 |
| 7 | 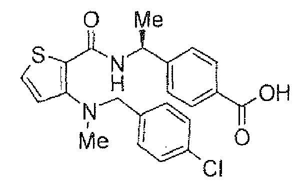 | | 8 | 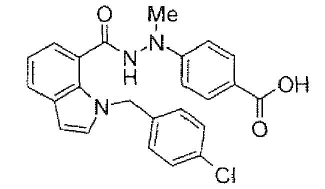 |

**[Table 13]**

| | | | | |
|---|---|---|---|---|
| 9 | 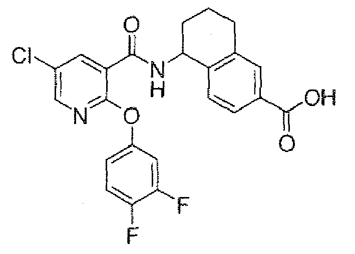 | | 10 | 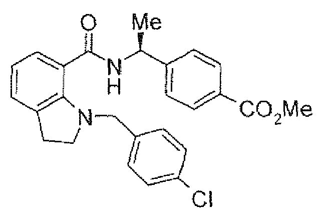 |
| 11 | 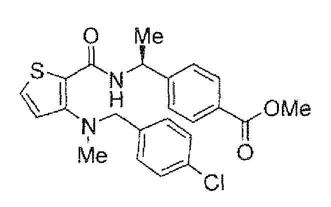 | | 12 | 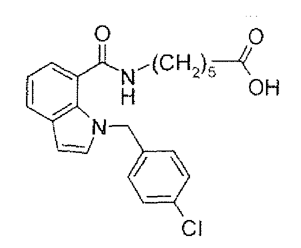 |
| 13 | 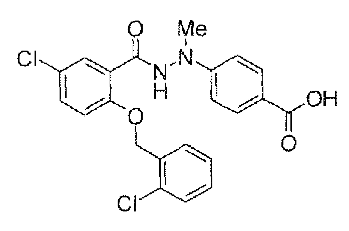 | | 14 | 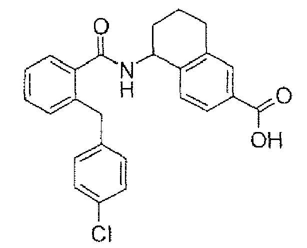 |
| 15 | 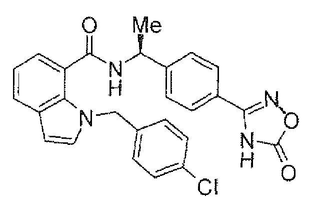 | | 16 | 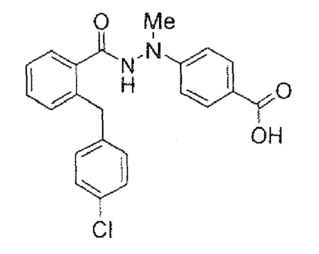 |

**[Table 14]**

| | | | | |
|---|---|---|---|---|
| 17 | 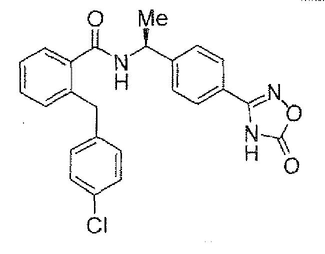 | | 18 | 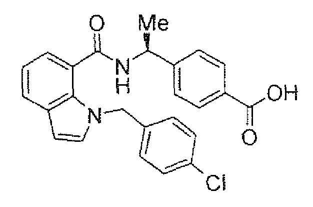 |
| 19 | 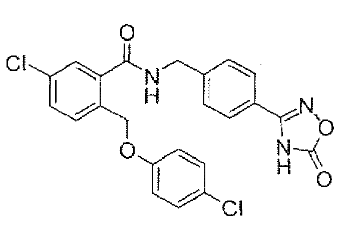 | | 20 | 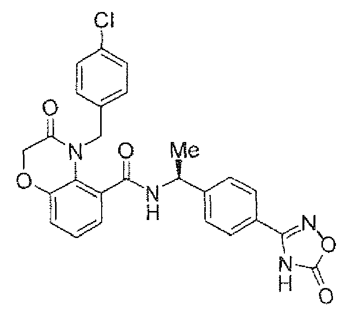 |
| 21 | 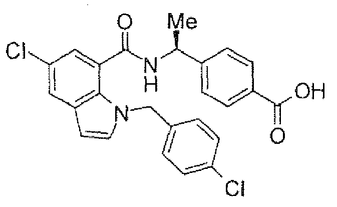 | | 22 | 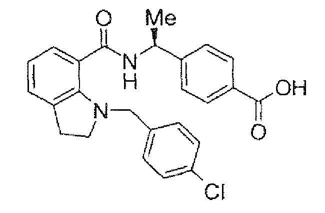 |
| 23 | 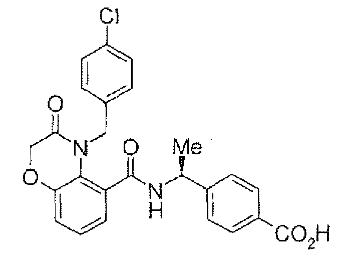 | | 24 | 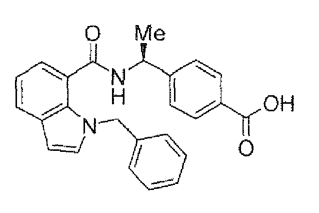 |

**[Table 15]**

| | | | | |
|---|---|---|---|---|
| 25 | 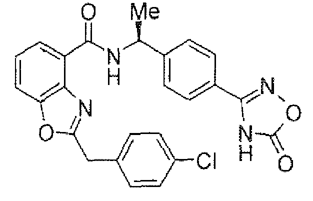 | | 26 | 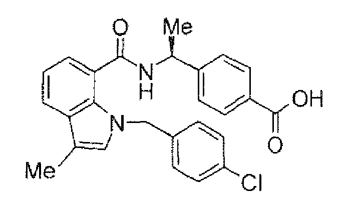 |
| 27 | 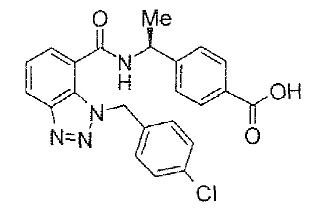 | | 28 | 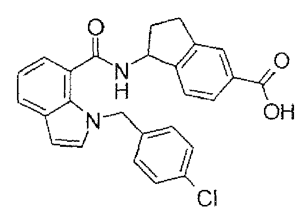 |
| 29 | 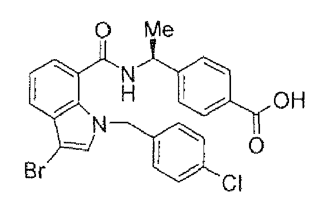 | | 30 | 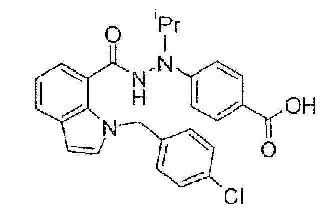 |
| 31 | 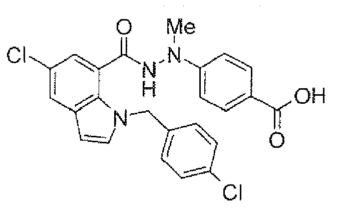 | | 32 | 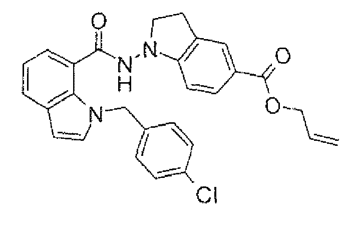 |

**[Table 16]**

| | | | | |
|---|---|---|---|---|
| 33 | 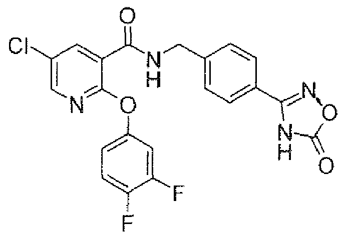 | | 34 | 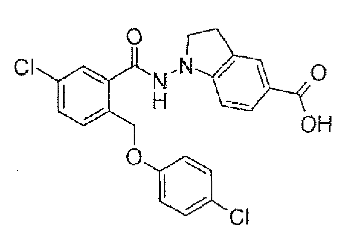 |
| 35 | 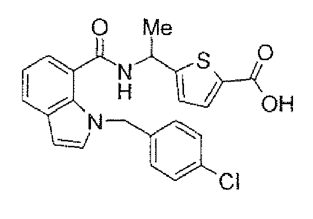 | | 36 | 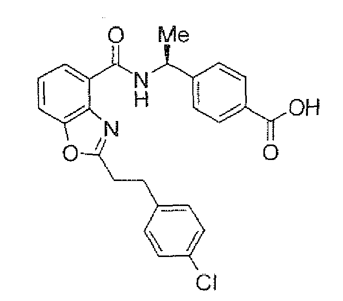 |
| 37 | 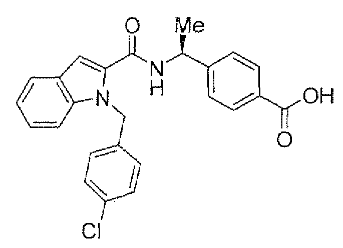 | | 38 | 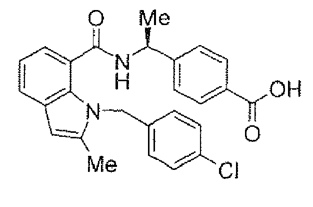 |
| 39 | 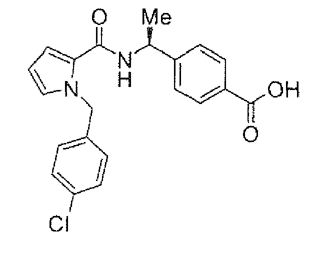 | | 40 | 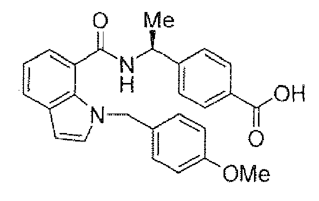 |

**[Table 17]**

| | | | | |
|---|---|---|---|---|
| 41 | 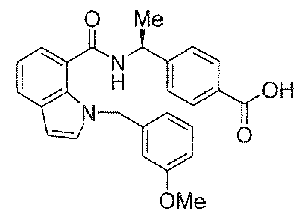 | | 42 | 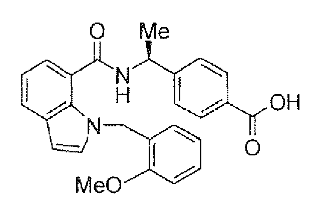 |
| 43 | 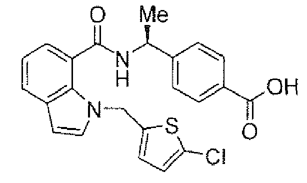 | | 44 | 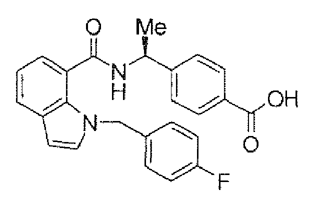 |
| 45 | 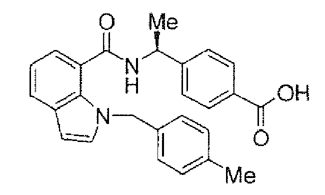 | | 46 | 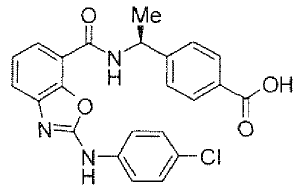 |
| 47 | 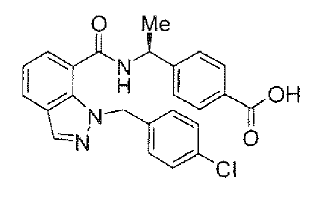 | | 48 | 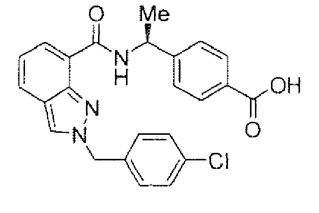 |

**[Table 18]**

| | | | | |
|---|---|---|---|---|
| 49 | 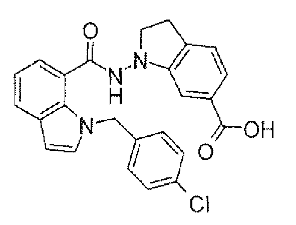 | | 50 | 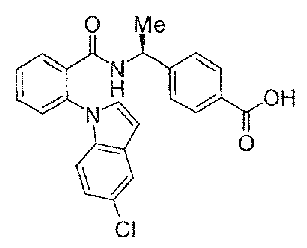 |
| 51 | 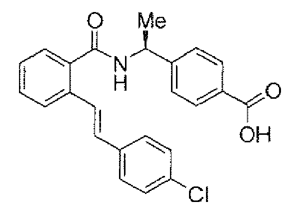 | | 52 | 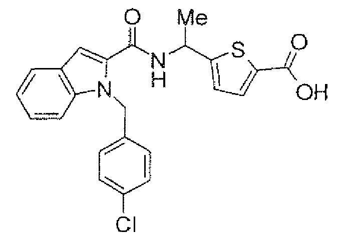 |
| 53 | 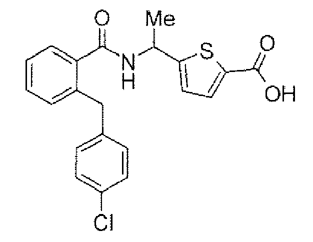 | | 54 | 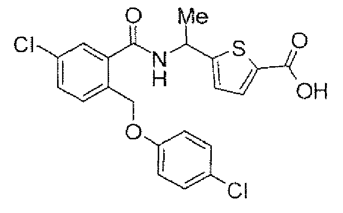 |
| 55 | 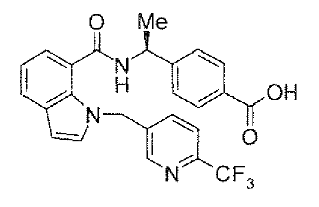 | | 56 | 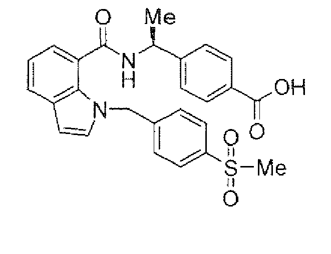 |

**[Table 19]**

| | | | | |
|---|---|---|---|---|
| 57 | 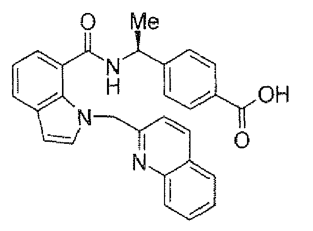 | | 58 | 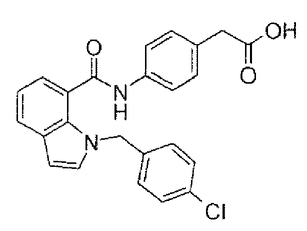 |
| 59 | 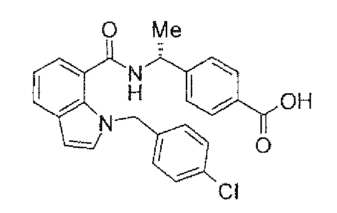 | | 60 | 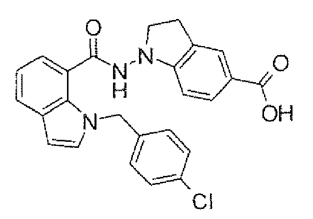 |
| 61 | 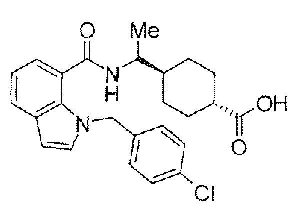 | | 62 | 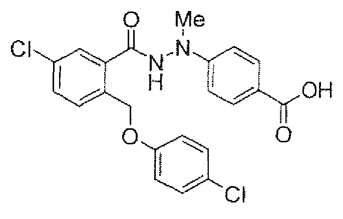 |
| 63 | 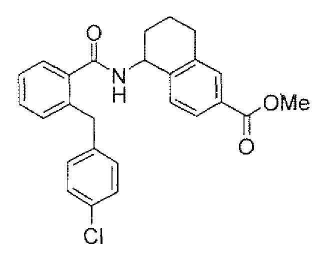 | | 64 | 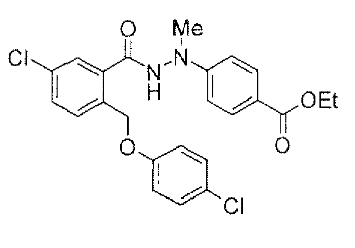 |

**[Table 20]**

| | | | | |
|---|---|---|---|---|
| 65 | 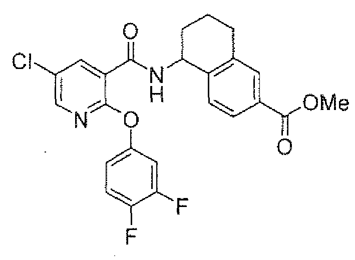 | | 66 | 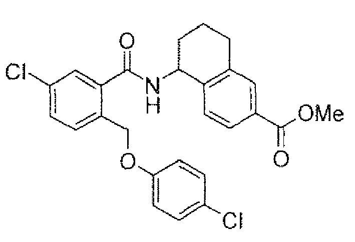 |
| 67 | 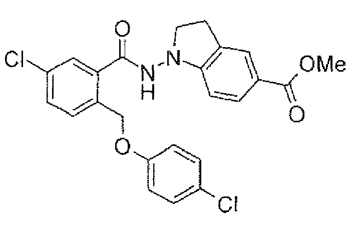 | | 68 | 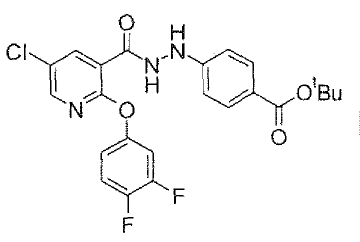 |
| 69 | 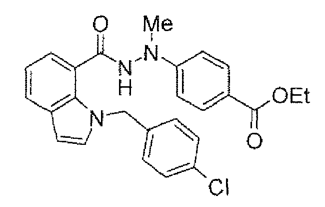 | | 70 | 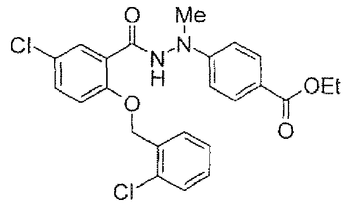 |
| 71 | 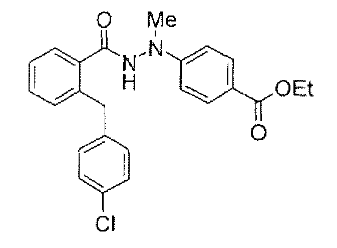 | | 72 | 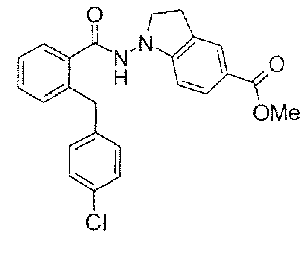 |

**[Table 21]**

| | | | | |
|---|---|---|---|---|
| 73 | 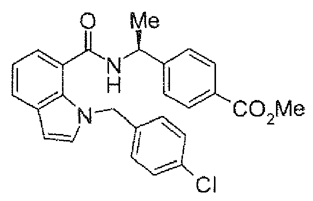 | | 74 | 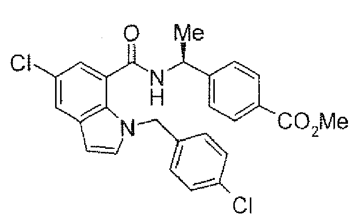 |
| 75 | 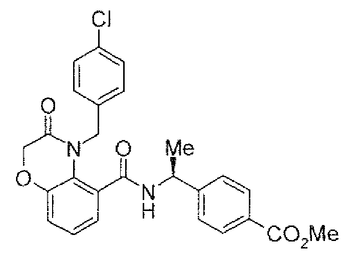 | | 76 | 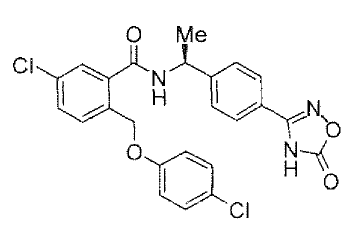 |
| 77 | 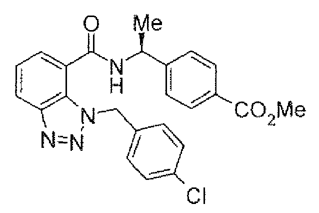 | | 78 | 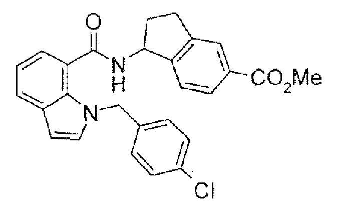 |
| 79 | 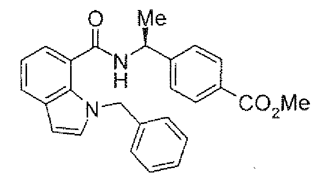 | | 80 | 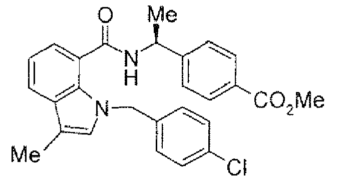 |
| 81 | 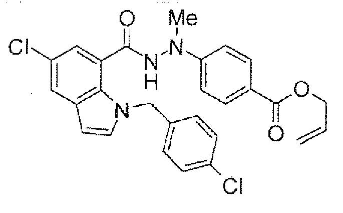 | | 82 | 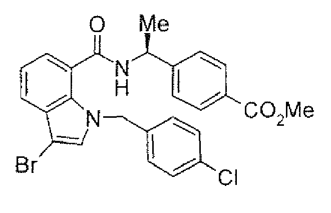 |

**[Table 22]**

| | | | | |
|---|---|---|---|---|
| 83 | 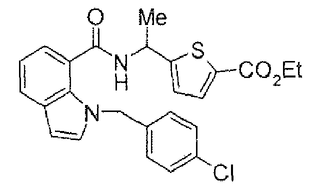 | | 84 | 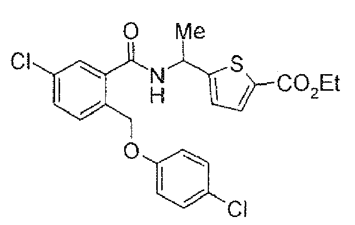 |
| 85 | 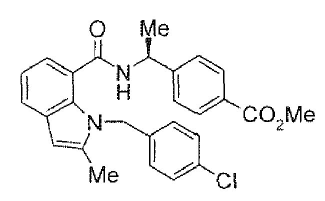 | | 86 | 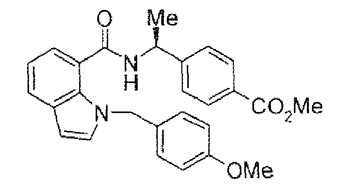 |
| 87 | 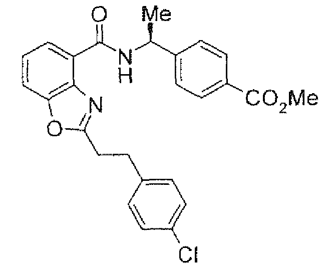 | | 88 | 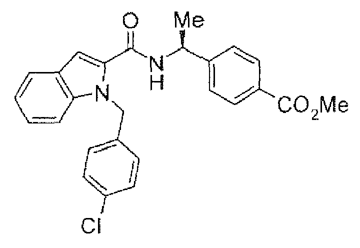 |
| 89 | 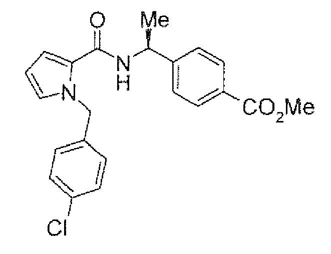 | | 90 | 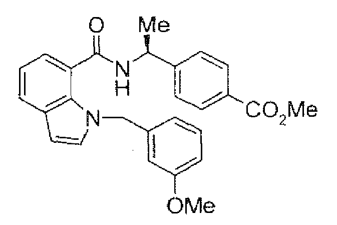 |

**[Table 23]**

| | | | | |
|---|---|---|---|---|
| 91 | 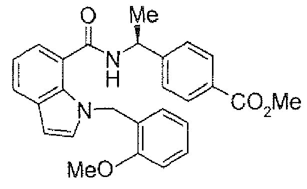 | | 92 | 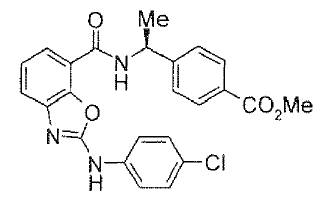 |
| 93 | 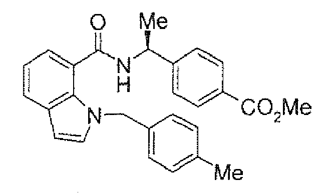 | | 94 | 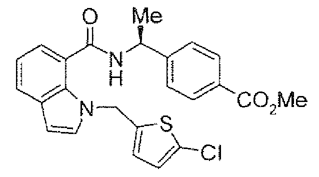 |
| 95 | 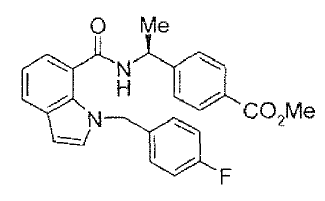 | | 96 | 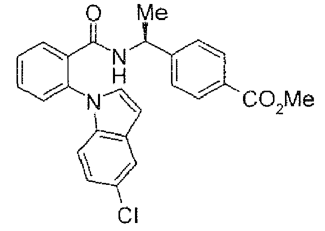 |
| 97 | 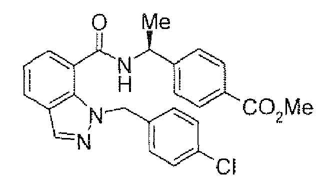 | | 98 | 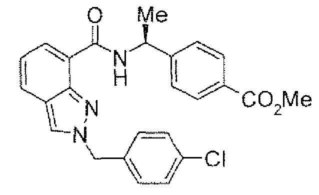 |
| 99 | 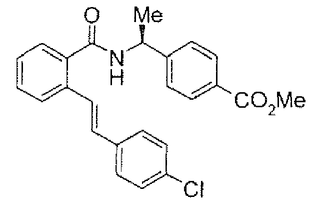 | | 100 | 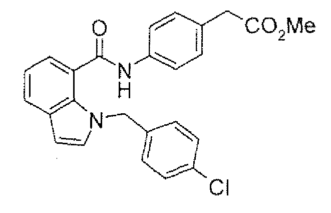 |

**[Table 24]**

| | | | | |
|---|---|---|---|---|
| 101 | 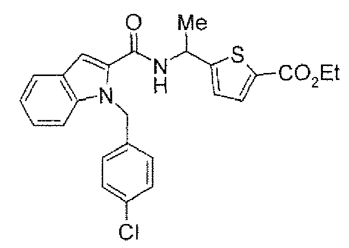 | | 102 | 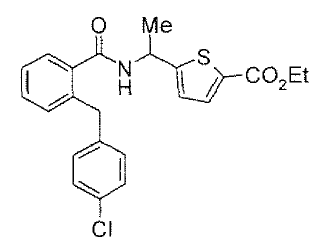 |
| 103 | 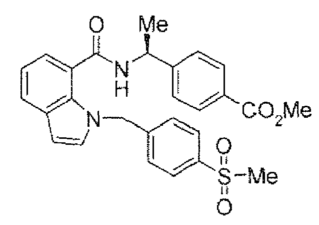 | | 104 | 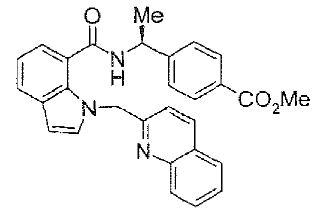 |
| 105 | 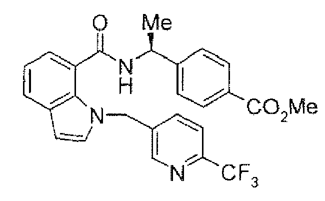 | | 106 | 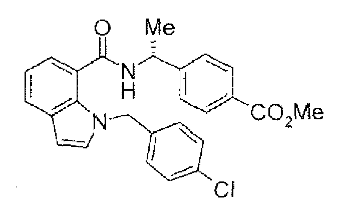 |
| 107 | 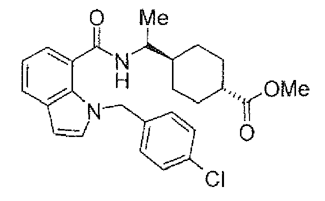 | | 108 | 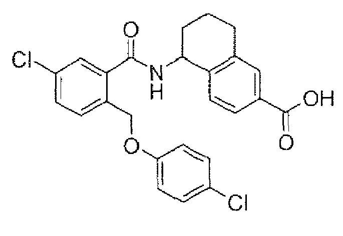 |

**[Table 25]**

| | | | | |
|---|---|---|---|---|
| 109 | 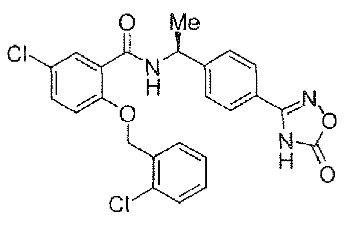 | | 110 | 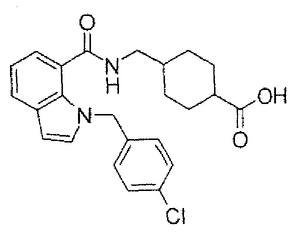 |
| 111 | 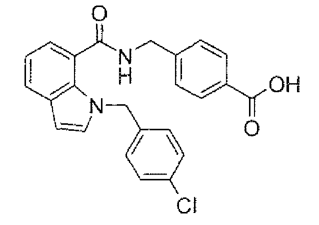 | | 112 | 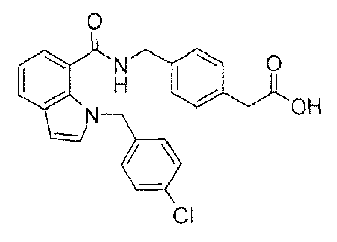 |
| 113 | 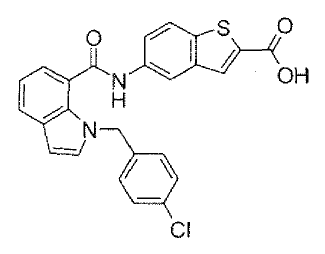 | | 114 | 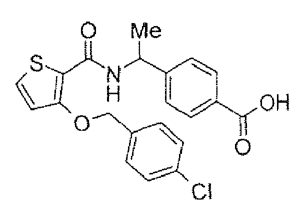 |

**[Table 26]**

| Ex | Syn | Dat |
|---|---|---|
| 1 | A | ESI+: 474 |
| 2 | B | NMR1: 12.4-12.3 (1H, brs), 10.8-10.7 (1H, brs), 8.39-8.31 (2H, m), 7.77 (2H, d, J = 7.8 Hz), 7.60-7.47 (2H, m), 7.20-7.13 (1H, m), 6.95 (2H, d, J = 7.8 Hz), 3.28 (3H, s)-., ESI+: 434 |
| 3 | C | ESI+: 420 |
| 4 | D | NMR1: 12.6-12.4 (1H, brs), 11.7 (1H, s), 8.26 (1H, s), 7.86-7.71 (2H, m), 7.59-7.31 (6H, m), 7.19 (2H, d. J = 8.4 Hz), 6.96 (1H, d, J = 8.5 Hz), 6.66 (1H, d, J = 3.4 Hz), 4.23 (2H, s); ESI+:405 |
| 5 | E | ESI+: 476 |
| 6 | F | ESI+: 486 |
| 7 | G | NMR1: 13.0-12.8 (1H, brs), 9.36 (1H, d, J = 7.8 Hz), 7.91 (2H, d, J =8.3 Hz), 7.68 (1H, d, J = 5.4 Hz), 7.43 (2H, d, J = 8.3 Hz), 7.34 (1H, d, J = 5.4 Hz), 7.29 (2H, d, J = 8.3 Hz), 7.20 (2H, d, J = 8.3 H), 5.17-5.05 (1H, m), 4.19-4.06 (2H, m). 2.64 (3H, s). 1.43 (3H, d, J = 6.8 Hz); ESI+: 429 |
| 8 | G | NMR1: 12.30 (1H, s), 10.65 (1H, s), 7.83 (1H, d, J = 8.0 Hz), 7.72 (2H, d, J = 9.1 Hz), 7.50 (1H, d, J = 3.3 Hz), 7.48 (1H, d, J = 7.1 Hz), 7.33 (2H, d, J = 8.4 Hz), 7.18 (1H, dd, J = 7.7, 8.0 Hz), 6.79 (2H, d, J = 8.4 Hz), 6.70 (2H, d, J = 9.1 Hz), 6.68 (1H, d, J = 3.3 Hz), 5.64 (2H, s), 2.99 (3H, s); ESI+: 434 |
| 9 | G | NMR1: 12.9-12.8 (1H, brs), 8.88 (1H, d, J = 8.7 Hz), 8.29 (1H, d, J = 2.6 Hz), 8.18 (1H, d, J = 2.6 Hz), 7.71-7.68 (1H, brs), 7.67-7.62 (1H, m), 7.54-7.37 (3H, m), 7.11-7.05 (1H, m), 5.27-5.18 (3H, m), 2.85-2.76 (2H, m), 2.09-1.98 (1H, m), 1.97-1.86 (1H, m), 1.84-1.73 (2H, m); ESI+: 459 |
| 10 | H | ESI+; 449 |
| 11 | I | ESI+: 443 |
| 12 | J | ESI+: 399 |
| 13 | G | NMR1: 12.36-12.22 (1H, brs), 10.44 (1H, s), 7.70-7.59 (4H, m), 7.58-7.52 (2H, m), 7.45-7.35 (2H, m), 7.32 (1H, d, J = 9.0 Hz), 6.79 (2H, d, J = 9.0 Hz), 5.30 (2H, s), 3.20 (3H, s); ESI-: 443 |
| 14 | G | NMR1: 12.8 (1H, s), 8.70 (1H, d, J = 8.7 Hz), 7.71-7.63 (2H, m), 7.42-7.17 (8H, m), 7.12 (1H, d, J = 7.9 Hz), 5.21-5.08 (1H, m), 4,27-4.10 (2H, m), 2.85-2.68 (2H, m), 1.99-1.84 (2H, m), 1.81-1.63 (2H, m); ESI+: 420 |

**[Table 27]**

| | | |
|---|---|---|
| 15 | A | NMR1: 13.10-12.79 (1H, brs), 8.85 (1H, d), 7.75 (2H, d, J = 8.2 Hz), 7.71 (1H, d, J = 7.8 Hz), 7.53 (2H, d, J = 8.5 Hz), 7.52 (1H, d, J = 3.0 Hz), 7.21 (2H, d, J = 8.2 Hz), 7.20 (1H, d, J = 7.4 Hz), 7.09 (1H, dd, J = 7.4. 7.8 Hz), 6.79 (2H, d, J = 8.5 Hz), 6.62 (1H, d, J = 3.0 Hz), 5.52 (1H, d, J = 16.2 Hz), 5.46 (1H, d, J = 16.2 Hz), 5.12-5.02 (1H, m), 1.27(3H, d, J = 7.0 Hz): ESI-: 471 |
| 16 | G | NMR1: 12.7-12.0 (1H, brs), 10.6 (1H, s), 7.76 (2H, d, J = 8.9 Hz), 7.61 (1H, d, J = 7.5 Hz), 7.53-7.27 (5H, m), 7.18 (2H, d, J = 8.4 Hz), 6.73 (2H. d, J = 9.0 Hz), 4.16 (2H, s), 3.15 (3H, s); FAB+: 395 |
| 17 | A | ESI-: 432 |
| 18 | G | NMR1: 12.88-12.78 (1H, brs), 8.84 (1H, d, J = 7.9 Hz), 7.88 (2H, d, J = 8.3 Hz) 7.70 (1H, d, J = 6.8 Hz), 7.52 (1H, d, J = 3.1 Hz), 7.44 (2H, d, J = 8.3 Hz), 7.23-7.15 (3H, m), 7.11-7.05 (1H, m), 6.78 (2H, d, J = 8.6 Hz), 6,61 (1H, d, J = 3.1 Hz), 5.51 (1H, d, J = 16.3 Hz). 4.46 (1H, d, J = 16.3 Hz), 5.12-5.02 (1H, m), 1.26 (3H. d, J = 7.0 Hz). FAB+: 433 |
| 19 | A | ESI-: 468 |
| 20 | A | FAB+: 505 |
| 21 | G | NMR1: 12.84 (1H, brs), 8.96 (1H, d, J = 7.5 Hz). 7.89 (2H, d, J = 8.4 Hz). 7.78 (1H, d, J = 2.0 Hz), 7.61 (1H, d, J = 3.4 Hz), 7.45 (2H, d, 3 = 8.4 Hz). 7,22-7-12 (3H, m), 6.76 (2H, d, J = 8.5 H), 6.62 (1H, d, J = 3.4 Hz), 5.47 (2H, s), 5.09-4.96 (1H, m). 1.27 (3H, d, J = 7.1 Hz); ESI+: 467 |
| 22 | G | NMR1: 12.79 (1H, brs), 8.86 (1H, d, J = 7.90 Hz), 7.79 (2H, d, J = 8.3 Hz), 7.40 (2H, d, J = 8.3 Hz), 7.30 (2H, d, J = 8.4 Hz), 7.20 (2H, d, J = 8.4 Hz), 7.14-7.09 (1H, m), 7.05-7.01 (1H, m), 6.65 (1H, t, J = 7.4 Hz), 5.08-4.97 (1H, m), 4.19 (1H, d, J = 15.3 Hz), 4.14 (1H, d, J = 15.3 Hz), 3.33-3.15 (2H, m), 2.91 (2H, t, J = 8.7 Hz), 1.31 (3H. d, J = 7.0 Hz); ESI+: 435 |
| 23 | G | ESI-; 463 |
| 24 | G | ESI-: 397 |
| 25 | A | ESI+: 475 |
| 26 | G | NMR1: 12.83 (1H, brs), 8.84 (1H, d, J = 7.9 Hz), 7.87 (2H d, J = 8.3 Hz), 7.64 (1H, dd, J = 1.2, 7.6 Hz), 7.44 (2H, d, J = 8.3 Hz), 7.29-7.26 (1H, m), 7.22-7.16 (3H, m). 7.08 (1H, t, J = 7.6 Hz), 6.79 (2H, d, J = 8.4 Hz), 5.43 (1H, d, J = 16.3 Hz), 5.37 (1H, d, J = 16.3 Hz), 5.12-5.02 (1H, m), 2.28 (3H, s), 1.27 (3H, d, J = 7.0 Hz); ESI+: 447 |
| 27 | G | NMR1: 12.87 (1H, brs), 9.21 (1H, d, J = 8.1 Hz), 8.25 (1H, d, J = 8.1 Hz), 7.92 (2H, d, J = 8.4 Hz), 7.75 (1H, dd, J = 0.9, 7.3 Hz), 7.55-7.43 (3H, m), 7.23-7.16 (2H, m), 6.91 (2H, d, J = 8.6 Hz), 6.11 (1H, d, J = 15.6 Hz), 6.00 (1H, d, J = 15.6 Hz), 5.23-5.13 (1H, m). 1.38 (3H. d, J = 7.0 Hz); ESI+: 435 |

**[Table 28]**

| | | |
|---|---|---|
| 28 | G | NMR1: 12.9-12.6 (1H, brs), 8.7 (1H, d, J =8.3 Hz), 7.78 (1H, s), 7.76-7.68 (2H, m), 7.56 (1H, d, J = 3.4 Hz), 7.30 (2H, d, J = 8.6 Hz), 7.20 (1H, d, J = 7.4 Hz), 7.07 (1H, t, J = 7.4 Hz), 6.99 (1H, d, J = 7.4 Hz), 6.87 (2H, d, J = 8.6Hz), 6.64 (1H, d, J = 3.4 Hz), 5.67 (2H, s), 5.48-5.36 (1H, m), 3.01-2.90 (1H, m), 2.90-2.78 (1H, m), 2.39-2.28 (1H, m), 1.83-1.70 (1H, m); ESI+: 445 |
| 29 | G | NMR1: 8.94 (1H, d, J = 7.8 Hz), 7.88 (2H, d, J = 8.3 Hz), 7.80 (1H, s), 7.58 (1H, dd. J = 1.0, 7.8 Hz), 7.44 (2H, d, J = 8.3 Hz), 7.33-7.29 (1H, m), 7.25-7.18 (3H, m), 6.83 (2H, d, J = 8.5 Hz), 5.51 (1H, d, J = 16.2 Hz) 5.4.5 (1H, d, J = 16.2 Hz.), 5.11-5.02 (1H, m), 1.28 (3H, d, J = 7.0 Hz); ESI+: 511 |
| 30 | G | NMR 1: 12.3-12.2 (1H, brs), 10.4 (1H, s), 7.88-7.84 (1H, m), 7.62 (2H, d, J = 9.0 Hz), 7.60-7.56 (1H, m), 7.42 (1H, d, J = 3.2 Hz), 7.34 (2H, d, J = 8.4 Hz), 7.24-7.19 (1H, m), 6.77 (2H, d, J = 8.4 Hz), 6.71 (1H, d, J = 3.2 Hz), 6.62 (2H, d, J = 9.0 Hz). 5.82-5.51 (2H, m), 4.35-4.26 (1H, m), 1.81-1.03 (6H, m): ESI+: 462 |
| 31 | B | NMR 1: 12.28 (1H, brs), 10.78 (1H, s), 7.91 (1H, d. J = 2.1 Hz), 7.72 (2H, d, J = 8.9 Hz), 7.58 (1H, d, J = 3.3 Hz), 7.46 (1H, d, J = 2.1 Hz), 7.34 (2H, d, J = 8.4 Hz). 6.78 (2H, d, J = 8.4 Hz), 6.70 (1H, d, J = 3.3 Hz), 6.67 (2H, d, J = 8.9 Hz), 5.61 (2H, s), 3.00 (3H, s): ESI+: 468 |
| 32 | A | ESI+: 486 |
| 33 | A | ESI+: 459 |
| 34 | G | NMR1: 12.55-12.25 (1H, br). 10.56 (1H, s), 7.76-7.56 (3H, m), 7.43-7.32 (2H, m), 7.10-6-98 (3H, m), 6.66 (2H, d, J = 8.2 Hz), 5.23 (2H, s), 3.71-3.62 (2H, m), 3.06-2.97 (2H, m); FAB+: 457 |
| 35 | G | NMR1: 12.96 (1H, brs), 8.94 (1H, d, J = 7.8 Hz), 7.72 (1H, dd, J = 1.0, 7.6 Hz). 7.59-7.54 (2H, m), 7.24 (2H, d, J = 8.3 Hz), 7.19 (1H, dd, J = 1.0, 7.4 Hz). 7.09 (1H, dd, J = 7.4, 7.6 Hz), 6.99 (1H, d, J = 3.9 Hz), 6.82 (2H, d, J = 8.3 Hz), 6.63 (1H, d, J = 2.9 Hz), 5.60 (1H, d, J = 16.5 Hz), 5.49 (1H, d, J = 16.5 Hz), 5.32-5.22 (1H, m), 1.34 (3H, d, J = 7.1 Hz); ESI+: 439 |
| 36 | G | ESI+: 449 |
| 37 | G | NMR1: 12.84 (1H, s), 9.02 (1H, d, J = 8.0 Hz), 7.89 (2H, d, J = 8.3 Hz), 7.68 (1H, d, J = 8.0 Hz), 7.53 (1H, d, J = 8.3 Hz), 7.46 (2H, d, J = 8.3 Hz), 7.30 (1H, s), 7.26 (2H, d, J = 8.4 Hz), 7.25-7.21 (1H, m), 7.14-7.08 (1H, m), 7.02 (2H, d, J = 8.4 Hz), 5.76 (2H, s), 5.22-5.12 (1H, m), 1.48 (3H, d, J = 7.1 Hz); ESI+: 433 |
| 38 | G | NMR1: 12.82 (1H, brs), 8.85 (1H, d, J = 7.9 Hz), 7.83 (2H, d, J = 8.2 Hz), 7.59 (1H, dd, J = 1.1, 7.5 Hz), 7.37 (2H, d, J = 8.2 Hz), 7.2 (2H, d, J = 8.5 Hz), 7.12-7.00 (2H, m), 6.66 (2H, d, J = 8.5 Hz), 6.44 (1H, s), 5.46 (1H, d, J = 17.8 Hz), 5.42 (1H, d, J = 17.8 Hz), 5,09-4,97 (1H, m), 2.31 (3H, s), 1.21 (3H, d, J = 7.1 Hz); ESI+: 447 |
| 39 | G | NMR1: 12.8-1.27 (1H, brs), 8.40 (1H, d, J = 7.9 Hz), 7.87 (2H, d. J = 8.4 Hz), 7.39 (2H, d, J = 8.4 Hz), 7.30 (2H, d, J = 8.5 Hz), 7.13-7.07 (1H, m), 7.05 (2H, d, J = 8.5 Hz), 6.97-6.90 (1H, m), 6.14-6.07 (1H, m), 5.54-5.45 (2H, m), 5.12-5.03 (1H, m) 1.42 (3H, d, J = 7.2 Hz): ESI+: 383 |

**[Table 29]**

| | | |
|---|---|---|
| 40 | G | NMR1: 12.84 (1H, brs), 8.90 (1H, d, J = 7.9 Hz), 7.89 (2H, d, J = 8.2 Hz), 7.68 (1H, dd, J = 1.0, 7.6 Hz), 7.53-7.43 (3H, m). 7.21 (1H, dd, J = 1.0, 7.4 Hz), 7.06 (1H, dd, J = 7.4, 7.6 Hz), 6.80 (2H, d, J = 8.6 Hz), 6.68 (2H, d, J = 8.6 Hz), 6.57 (1H, d, J = 3.3 Hz), 5.42 (1H, d, J = 15.6 Hz), 5.36 (1H, d, J = 15.6 Hz), 5.21-5.11 (1H, m), 3.65 (3H, s), 1.34 (3H, d, J = 7.1 Hz); ESI+: 429 |
| 41 | G | NMR1: 12.82 (1H, brs), 8.88 (1H, d, J = 7.8 Hz), 7.86 (2H, d, J = 8.3 Hz), 7.70 (1H, d, J = 7.9 Hz), 7.50 (1H, d, J = 3.2 Hz), 7.44 (2H, d, J = 8.3 Hz), 7.20 (1H, d, J = 7.1 Hz), 7.12-7.04 (2H, m), 6.76 (1H, dd, J = 2.4, 8.2 Hz), 6.60 (1H, d, J = 3.2 Hz), 6.41 (1H, s), 6.36 (1H, d, J = 7.7 Hz), 5.53 (1H, d, J = 16.2 Hz), 5.42 (1H, d, J = 16.2 Hz), 5.14-5.04 (1H, m), 3.64 (3H, s), 1.26 (3H, d. J = 7.0 Hz); FAB+: 429 |
| 42 | G | NMR1: 12.81 (1H, brs), 8.85 (1H, d, J = 7.9 Hz), 7.81 (2H, d, J = 8.1 Hz). 7.71 (1H, d, J = 7.8 Hz), 7.39-7.32 (3H, m), 7.24-7.15 (2H, m), 7.09 (1H, dd, J = 7.5, 7.8 Hz), 6.96 (1H, d, J = 8.2 Hz), 6.68 (1H, t, J = 7.4 Hz), 6.59 (1H, d, J = 3.1 Hz), 6.10 (1H, d, J = 7.7 Hz), 5.52 (1H, d, J = 17.0 Hz), 5.36 (1H, d, J = 17.0 Hz), 5.05-4.95 (1H, m), 3.79 (3H, s), 1.20 (3H, d, J = 7.0 Hz); ESI+: 429 |
| 43 | G | NMR1: 12.86 (1H, brs), 9.08 (1H, d, J = 7.8 Hz), 7.95-7.90 (2H, m), 7.70 (1H, d, J = 7.9 Hz), 7.57-7.49 (3H, m), 7.29 (1H, d, J = 7.2 Hz), 7.14-7.07 (1H, m), 6.77 (1H, dd, J = 1.4, 3.9 Hz), 6.61-6.58 (1H, m), 6.47-6.43 (1H, m), 5.66 (1H, d, J = 16.1 Hz), 5.58 (1H, d, J = 16.1 Hz), 5.28-5.18 (1H, m), 1.43 (3H, d, J = 7.0 Hz); ESI+: 439 |
| 44 | G | NMR1: 12.85 (1H, brs), 8.89 (1H, d, J = 7.8 Hz), 7.88 (2H, d, J = 7.9 Hz), 7.70 (1H, d, J = 7.8 Hz), 7.52 (1H, d, J = 3.0 Hz), 7.46 (2H, d, J = 7.9 Hz), 7.21 (1H, d, J = 7.3 Hz), 7.08 (1H, dd, J = 7.3, 7.8 Hz), 7.01-6.93 (2H, m), 6.88-6.81 (2H, m). 6.61 (1H, d, J = 3.0 Hz), 5.51 (1H, d, J = 16.0 Hz), 5.43 (1H, d, J = 16.0 Hz), 5.15-5.05 (1H, m), 1.28 (3H, d, J = 7.1 Hz); ESI+: 417 |
| 45 | G | NMR1: 12.84 (1H, brs), 8.88 (1H, d, J = 7.9 Hz), 7.90-7.83 (2H, m), 7.68 (1H, d, J = 7.9 Hz), 7.51-7.4 (3H, m), 7.19 (1H, d, J = 6.8 Hz), 7.10-7.03 (1H, m), 6.94 (2H, d, J = 7.4 Hz), 6.73 (2H, d, J = 7.4 Hz), 6.60-6.57 (1H, m), 5.47 (1H, d, J = 16.0 Hz), 5.40 (1H, d, J = 16.4 Hz), 5.17-5.07 (1H, m), 2.19 (3H, s), 1.30 (3H, d, J = 6.9 Hz); ESI+: 413 |
| 46 | G | NMR1: 12.98-12.75 (1H, br), 10.97 (1H, s), 8.85 (1H, d, J = 7.6 Hz), 7.93 (2H, d, J = 8.3 Hz), 7.80 (2H, d, J = 8.8 Hz), 7.60 (1H, d, J = 6.8 Hz), 7.55 (2H, d, J = 8.3 Hz). 7.48-7.40 (3H, m), 7.34-7.28 (1H, m), 5.29-5.17 (1H, m), 1.53 (3H, d, J = 7.1 Hz); ESI+: 436 |
| 47 | G | NMR1: 12.85 (1H, brs), 9.10 (1H, d, J = 7.8 Hz), 8.26 (1H, d, J = 1.5 Hz), 7.94 (1H, d, J = 8.3 Hz), 7.92-7.87 (2H, m), 7.55-7.44 (3H, m), 7.26-7.15 (3H, m), 6.84 (2H, d, J = 7.5 Hz), 5.72 (1H, d, J = 16.3 Hz), 5.68 (1H, d, J = 16.3 Hz), 5.21-5.11 (1H, m), 1.35 (3H, d, J = 6.8 Hz); ESI+: 434 |
| 48 | G | NMR1: 12.90 (1H, brs), 9.58 (1H, d, J = 7.8 Hz), 8.79 (1H, s), 8.03-7.88 (4H, m), 7.55-7.40 (6H, m), 7.24-7.18 (1H, m), 5.80-5.70 (2H, m), 5.31-5.22 (1H, m), 1.52 (3H, d, J = 7.0 Hz): ESI+: 434 |
| 49 | B | NMR1: 12.8-12.7 (1H, brs), 10.3 (1H, s), 7.79 (1H, d, J = 8.0 Hz), 7.56 (1H, d, J = 3.3 Hz), 7.44-7.28 (4H, m), 7.25-7.08 (3H, m), 6.92 (2H, d, J = 8.5 Hz), 6.67 (1H, d, J = 3.2 Hz), 5.59 (2H, s), 3.45 (2H, t, J = 8.1 Hz), 3.02 (2H, t, J = 8.1 Hz); ESI+: 446 |

**[Table 30]**

| | | |
|---|---|---|
| 50 | G | NMR1: 12.95-12.65 (1H, brs), 8.67 (1H, d, J = 7.9 Hz), 7.75 (2H, d, J = 6.6 Hz), 7.68-7.61 (3H, m), 7.57 (1H, dt, J = 1.5, 7.4 Hz), 7.49 (1H, dd, J = 2.3, 7.6 Hz), 7.45 (1H, d, J = 3.4 Hz), 7.19-7.09 (3H, m), 7.05 (1H, dd, J = 1.9, 8.8 Hz), 6.57 (1H, d, J = 2.6 Hz), 4.88-4.75 (1H, m), 1.14 (3H, d, J = 7.0 Hz); ESI+: 419 |
| 51 | G | NMR1: 13.1-12.8 (1H, brs), 9.02 (1H, d, J = 7.8 Hz), 7.93 (2H, d, J = 8.0 Hz), 7.83 (1H, d, J = 7.8 Hz), 7.55 (2H, d, J = 8.0 Hz), 7.46 (1H, dt, J = 1.4, 7.4 Hz), 7.43-7.3 (4H, m), 7.28 (2H, d, J = 8.8 Hz), 7.20 (1H, d, J = 16.4 Hz), 7.07 (1H, d, J = 16.4 Hz), 5.25-5.16 (1H, m), 1.46 (3H, d, J = 7.12 Hz); ESI+: 406 |
| 52 | G | NMR1: 9.16 (1H, d, J = 8.3 Hz), 7.69 (1H, d, J = 7.7 Hz), 7.60-7.53 (2H, m), 7.34-7.21 (4H, m), 7.16-7.07 (3H, m), 7.03-6.99 (1H, m), 5.87-5-76 (2H, m), 5.44-5.33 (1H, m), 1.58 (3H, d, J = 6.9 Hz); ESI+: 439 |
| 53 | G | NMR1: 8.98 (1H, d, J = 8.2 Hz), 7.60-7.56 (1H, m), 7.42-7.24 (6H, m), 7.21-7.14 (2H, m), 7.03-7.00 (1H, m), 5.40-5.29 (1H, m), 4.09 (2H, s), 1.47 (3H, d, J = 6.9 Hz); ESI+: 400 |
| 54 | G | NMR1: 9.20 (1H, d, J = 7.8 Hz), 7.63-7.52 (4H, m), 7.33-7.27 (2H, m), 7.07 (1H, dd, J = 1.0, 3.9 Hz), 6.95-6.90 (2H, m), 5.38-5.27 (1H, m), 5.24-5.15 (2H, m), 1.51 (3H, d, J = 7.5 Hz); ESI+: 450 |
| 55 | G | NMR1: 12.84 (1H, brs), 8.87 (1H, d, J = 7.6 Hz), 8.19 (1H, d, J = 1.6 Hz), 7.87 (2H, d, J = 8.5 Hz), 7.78-7.58 (3H, m), 7.43 (2H, d, J = 8.5 Hz), 7.36-7.09 (3H, m), 6.68 (1H, d, J = 3.4 Hz), 5.70 (1H, d, J = 17.3 Hz), 5.63 (1H, d. J = 17.3 Hz), 5.06-4.96 (1H, m), 1.20 (3H, d, J = 7.3 Hz); ESI+: 468 |
| 56 | G | NMR1: 12.85 (1H, brs), 8.86 (1H, d, J = 7.6 Hz), 7.87 (2H, d, J = 8.3 Hz), 7.77-7.70 (3H, m), 7.55 (1H, d, J = 3.1 Hz), 7.45 (2H, d, J = 8.3 Hz), 7.21 (1H, dd, J = 1.0, 7.6 Hz), 7.10 (1H, t, J = 7.6 Hz), 7.00 (2H, d, J = 8.4 Hz), 6.65 (1H, d, J = 3.1 Hz), 5.67 (1H, d, J = 16.8 Hz), 5.56 (1H, d, J = 16.8 Hz), 5.07-4.97 (1H, m), 3.13 (3H, s), 1.19 (3H, d, J = 6.9 Hz); ESI+: 477 |
| 57 | G | NMR1: 12.78 (1H, brs), 8.76 (1H, d, J = 7.9 Hz), 8.14 (1H, d, J = 8.5 Hz), 7.92-7.85 (2H, m), 7.78-7,68 (4H, m), 7.59-7.51 (2H, m), 7.32 (2H, d, J = 8.2 Hz), 7.21-7.08 (2H, m), 6.67 (1H, d, J = 3.2 Hz), 6.60 (1H, d, J = 8.5 Hz), 5.83 (1H, d, J = 17.0 Hz), 5.75 (1H, d, J = 17.0 Hz), 4.99-4.89 (1H, m), 0.96 (3H, d, J = 7.0 Hz); ESI+: 450 |
| 58 | G | NMR1: 12.3-12.2 (1H, br.s), 10.2 (1H, s), 7.74 (1H, d, J = 7.9 Hz), 7.62 (1H, d, J = 3.3 Hz), 7.57 (2H, d, J = 8.2 Hz), 7.29-7.18 (3H, m), 7.15-7.05 (3H, m), 6.82 (2H, d, J = 8.2 Hz), 6.65 (1H, d, J = 3.3 Hz). 5.50 (2H, s), 3.55 (2H, s); ESI+: 419 |
| 59 | G | ESI+: 433 |
| 60 | B | NMR1: 12.3 (1H, s), 10.3 (1H, s), 7.81 (1H, d, J = 7.6 Hz), 7.67-7.59 (2H, m), 7.54 (1H, d, J = 3.3 Hz), 7.42 (1H, d, J = 6.8 Hz), 7.34 (2H, d, J = 8.5 Hz), 7.15 (1H, dd, J = 7.6, 7.6 Hz), 6.82 (2H, d, J = 8.4 Hz), 6.69 (1H, d, J = 3.1 Hz), 6.21 (1H, d, J = 8.1 Hz), 5.63 (2H, s), 3.50 (2H, t, J = 8.2 Hz), 3.00 (2H, t, J = 8.2 Hz); ESI+: 446 |
| 61 | G | NMR1: 12.0-11.8 (1H, brs), 8.15 (1H, d, J = 8.5 Hz), 7.69 (1H, d, J = 7.8 Hz), 7.48 (1H, d, J = 3.3 Hz), 7.26 (2H, d, J = 8.4 Hz), 7.15 (1H, d, J = 7.2 Hz), 7.10-7.03 (1H, m), 6.84 (2H, d, J = 8.4 Hz), 6.62 (1H, d, J = 3.3 Hz), 5.67-5.53 (2H, m, J = 2 Hz), 3.77-3.63 (1H, m), 2.11-2.00 (1H, m), 1.92-1.79 (2H, m), 1.75-1.58 (2H, m), 1.31-1.10 (3H, m), 0.99-0.92 (5H, m); ESI+: 439 |

**[Table 31]**

| | | |
|---|---|---|
| 62 | G | NMR1: 12.30 (1H, s), 10.85 (1H, s), 7.79 (1H, s), 7.71 (2H,d,J = 9.0 Hz), 7.68-7.61 (2H, m), 7.35 (2H, d, J = 9.0 Hz), 7.02 (2H, d, J = 9.0 Hz), 6.86 (2H, d, J = 9.0 Hz), 5.23 (2H, s), 3.23 (3H, s); ESI+: 445 |
| 63 | A | FAB+: 434 |
| 64 | A | ESI+: 473 |
| 65 | A | ESI+: 473 |
| 66 | A | ESI+: 484 |
| 67 | A | ESI+: 471 |
| 68 | A | ESI+: 476 |
| 69 | A | ESI+: 462 |
| 70 | A | ESI+: 473 |
| 71 | A | FAB+: 423 |
| 72 | A | FAB+: 421 |
| 73 | A | ESI+: 447 |
| 74 | A | ESI+: 481 |
| 75 | A | ESI+: 479 |
| 76 | A | NMR1: 12.98-12.85 (1H, brs), 9.06 (1H, d, J = 7.8 Hz), 7.72 (2H, d, J = 8.4 Hz), 7.62-7.52 (5H, m), 7.27 (2H, d, J = 8.8 Hz), 6.85 (2H, d, J = 8.8 Hz), 5.18-5.05 (3H, m), 1.44 (3H, d, J = 7.1 Hz); ESI-: 482 |
| 77 | A | ESI+: 449 |
| 78 | A | ESI+: 459 |
| 79 | A | ESI+: 413 |
| 80 | A | ESI+: 461 |
| 81 | A | FAB+: 508 |
| 82 | A | ESI+: 525 |
| 83 | A | ESI+: 467 |
| 84 | A | ESI+: 478 |
| 85 | A | ESI+: 461 |
| 86 | A | ESI+: 443 |
| 87 | A | ESI-: 463 |
| 88 | A | ESI: 447 |
| 89 | A | ESI+: 397 |

**[Table 32]**

| | | |
|---|---|---|
| 90 | A | ESI+: 443 |
| 91 | A | ESI+: 443 |
| 92 | A | ESI+: 450 |
| 93 | A | ESI+: 427 |
| 94 | A | ESI+: 453 |
| 95 | A | ESI+: 431 |
| 96 | A | FAB+: 433 |
| 97 | A | ESI+: 448 |
| 98 | A | ESI+: 448 |
| 99 | A | FAB+: 420 |
| 100 | A | ESI+: 433 |
| 101 | A | ESI+: 467 |
| 102 | A | ESI+: 428 |
| 103 | A | ESI+: 491 |
| 104 | A | ESI+: 464 |
| 105 | A | ESI+: 482 |
| 106 | A | ESI+: 447 |
| 107 | A | ESI+: 453 |
| 108 | G | NMRI : 8.99-8.94 (1H, m), 7.68 (1H, s), 7.62 (1H, d, J = 9.3 Hz), 7.59-7.53 (3H, m), 7.35 (1H, d, J = 7.6 Hz), 7.34 (2H, d, J = 9.0 Hz), 6.99 (2H, d, J = 9.0 Hz), 5.29 (1H, d, J= 12.0 Hz), 5.23 (1H, d, J = 12.0 Hz), 5.21-5.12 (1H, m), 2.85-2.73 (2H, m), 2.00-1.85 (2H, m), 1.83-1.69 (2H, m); ESI-: 468 |
| 109 | A | NMRI: 13.02-12.82 (1H, brs), 8.51 (1H, d, J = 7.6 Hz), 7.70-7.61 (4H, m), 7.59-7.52 (2H, m), 7.46-7.33 (5H, m), 5.29 (2H, s). 5.13-5.04 (1H, m), 1.27 (3H, d, J = 7.1 Hz); ESI-: 482 |
| 110 | J | ESI+: 425 |
| 111 | J | ESI+: 419 |
| 112 | J | ESI+: 433 |
| 113 | J | ESI+: 461 |
| 114 | J | ESI+: 416 |

### Sequence Listing Free Text

For the number title <400> in the following sequence listing, the nucleotide sequence of the rat EP4 (sequence number 1) is described.

## Claims

1. A compound of the formula (I) or a pharmaceutically acceptable salt thereof: 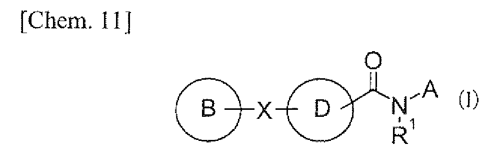 (wherein the symbols in the formula have the following meanings:
Ring B and Ring D: the same as or different from each other, each representing aryl which may be substituted, or a heterocycle which may be substituted,
X: single bond, -O-, -S-, -NH-, -N(R⁰)-, -N(R⁰)-R⁰⁰-, -O-R⁰⁰-, -R⁰⁰-O-, -R⁰⁰-, or -(lower alkenylene)-,
R⁰: lower alkyl,
R⁰⁰: lower alkylene,
R¹; H or R⁰,
A: -Z-R³, or a group represented by any one of the formulae (II) to (VII): 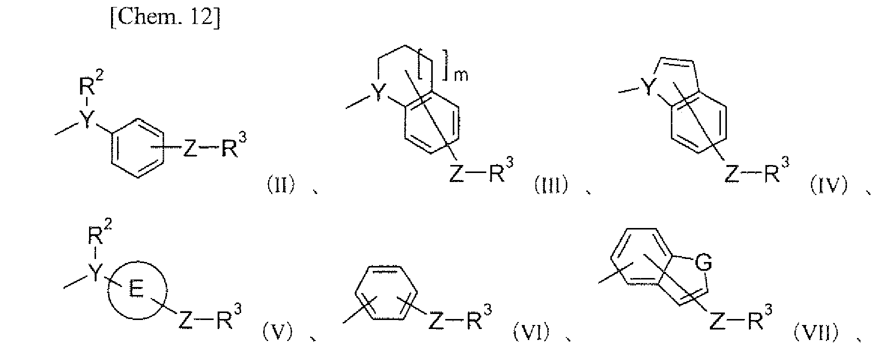
Ring E: a heterocycle or cycloalkanediyl which may be substituted,
Y: CH or N,
R²: H or R⁰,
m: 0 or 1,
Z: single bond or R⁰⁰,
provided that when A is -Z-R³, Z is R⁰⁰,
G: O, S, -N(R²)-, or -(HC=CH)-, and
R³: -CO₂H or a biological equivalent thereof,
provided that the compounds, in which, when D is phenyl or pyridyl which may be substituted, A is a group represented by the above formula (II), Y is CH, and Z is a single bond, R³ is -CO₂H, tetrazole, sulfonamide, or a carboxylate, are excluded).

2. A compound according to claim 1, wherein Ring D is a bicyclic heterocycle which may be substituted with halogen or R⁰, A is a group represented by the formula (II) or the formula (V), Y is CH, and R² is H, or a pharmaceutically acceptable salt thereof.

3. A compound according to claim 1, wherein Ring D is indolyl which may be substituted with halogen or R⁰, A is a group represented by the formula (II) or the formula (V), Y is CH, and R² is H, or a pharmaceutically acceptable salt thereof.

4. A compound according to claim 1, wherein Ring D is indolyl which may be substituted with halogen or R⁰, and Ring E is cyclohexanediyl, or a pharmaceutically acceptable salt thereof.

5. A compound according to claim 1, wherein Ring D is indole-1,2-diyl or pyrrole-1,2-diyl, which may be substituted with halogen or R⁰, or a pharmaceutically acceptable salt thereof.

6. A compound according to claim 1, wherein A is a group represented by any one of the formulae (II) to (V), and Y is N, or a pharmaceutically acceptable salt thereof.

7. A pharmaceutical composition comprising the compound according to claim 1 or a pharmaceutically acceptable salt thereof, and a pharmaceutically acceptable excipient.

8. A pharmaceutical composition for preventing or treating chronic renal insufficiency or diabetic nephropathy, which comprises the compound according to claim 1 or a pharmaceutically acceptable salt thereof.

9. Use of the compound according to claim 1 or a pharmaceutically acceptable salt thereof, for the manufacture of an agent for preventing or treating chronic renal insufficiency or diabetic nephropathy.

10. A method for preventing or treating chronic renal insufficiency or diabetic nephropathy, which comprises administering to a patient an effective amount of the compound according to claim 1 or a pharmaceutically acceptable salt thereof.


SEQUENCE LISTING

<110>  Astellas Pharma Inc.

<120>  Amide compounds

<130>  A08014

<160>  1

<210>  1

<211>  1501

<212>  DNA

<213>  Rat

<400>  1
aagctgtgta ctactgacca ccatcatgtc catccccgga gtcaacgcgt ccttctcctc     60
cactccggag aggttgaaca gcccagtgac cattcccgca gtgatgttta tcttcggggt    120
ggtgggcaac ctggtggcca tcgtagtatt gtgcaagtcg cgcaaggagc agaaggagac    180
taccttttac actctggtat gtgggctggc tgtcactgac ctactgggca cattgttggt    240
aagcccagtg accatcgcca catacatgaa gggccagtgg cccggagacc aggcattgtg    300
tgactacagc accttcatcc tacttttctt cggcctgtcg ggtctcagca tcatctgtgc    360
catgagcatt gagcgctacc tggccatcaa ccacgcctac ttctacagcc actacgtgga    420
caagcggctg gccggtctca cgctcttcgc cgtctatgca tctaacgtgc tcttctgcgc    480
actgcccaac atgggcctgg gtaggtccga gcggcagtac ccggggacct ggtgcttcat    540
cgactggacc accaacgtaa cggcctacgc cgccttctct tacatgtacg cgggcttcag    600
ttccttcctc atcctcgcca ccgtgctctg caatgtgctg gtgtgcggcg cgctgctccg    660
catgctccgc cagttcatgc gccgcacctc gctgggcacg gagcagcacc acgcggccgc    720
tgcagcagcg gtggcttcgg tggcctgtcg gggtcacgcg gccgcctccc cagccctgca    780
gcgcctcagt gactttcgcc gccgcaggag cttccggcgc atcgcgggtg cagagatcca    840
gatggtcatc ttactcatcg ccacctctct ggtggtgctc atctgctcca ttccgctcgt    900
ggtgcgagtg ttcatcaacc agttatatca gccaagtgtg gtgaaagaca tcagcagaaa    960
cccggatttg caggccatca gaattgcttc tgtgaacccc atcctggacc cttggatcta   1020
catccttctt cggaagactg tgctcagtaa agccatagaa aagatcaagt gcctcttctg   1080
ccgcattggt ggttctggca gagacggttc agcacagcac tgctcagaga gtcggaggac   1140
atcttctgcc atgtctggcc actcccgctc cttcctctcg cgggagttga gggagatcag   1200
cagcacctct cacaccctcc tatacctgcc agacctaact gaaagcagcc tcggaggcaa   1260
gaatttgctt ccaggtacgc atggcatggg cctgacccaa gcagacacca cctcgctgag   1320
aactttgcga atttcagaga cctcagactc ctcccagggc caggactctg agagtgtctt   1380
gttggtggat gaggttagtg ggagccagag agaggagcct gcctctaagg ggaactctct   1440
gcaagtcacg ttccccagtg aaacgctgaa attatctgaa aaatgtatat agtagcttaa   1500
a                                                                   1501